# EUROPEAN PATENT SPECIFICATION

(11) **EP 1 335 926 B1**
(45) Date of publication and mention of the grant of the patent: **13.06.2007**
(21) Application number: 01997500.2
(22) Date of filing: 20.11.2001
(51) Int. Cl.: C07H 1/08, C12N 15/10, A61K 31/711

(54) **METHOD OF SEPARATING EXTRA-CHROMOSOMAL DNA FROM OTHER CELLULAR COMPONENTS**
VERFAHREN ZUR ABTRENNUNG VON EXTRACHROMOSOMALER DNS AUS EINER ZELLSUSPENSION
PROCEDE POUR SEPARER L'ADN EXTRACHROMOSOMIQUE D'AUTRES COMPOSANTS CELLULAIRES

(30) Priority: 21.11.2000 GB 0028361
(43) Date of publication of application: 20.08.2003
(73) Proprietor: GLAXO GROUP LIMITED, Greenford, Middlesex UB6 0NN (GB)
(72) Inventor: CHARLTON, Henry, Beckenham, Kent BR3 3BS (GB); EON-DUVAL, Alex, Beckenham, Kent BR3 3BS (GB); MacDUFF, Robert, Beckenham, Kent BR3 3BS (GB)
(74) Representative: Bor, Fiona Rachel
(86) International application number: PCT/GB2001/005114
(87) International publication number: WO 2002/042317

(56) References cited:
- WO-A-00/09680
- WO-A-01/07599
- WO-A-98/30685
- KAHN DAVID W ET AL: "Purification of plasmid DNA by tangential flow filtration" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 69, no. 1, 5 July 2000 (2000-07-05), pages 101-106, XP002151908 ISSN: 0006-3592 cited in the application

## Description

### FIELD OF THE INVENTION

The present invention relates to a method of separating extra-chromosomal DNA from other cellular components, for example, RNA, genomic DNA, proteins and endotoxins. More particularly it relates to a method for producing pharmaceutical quality extra-chromosomal DNA without subjecting the extra-chromosomal DNA to an RNA digest. The invention also relates to extra-chromosomal DNA produced by the method and pharmaceuticals derived from such extra-chromosomal DNA, for example, DNA vaccines or gene therapy products. The invention also relates to a method of generating extra-chromosomal DNA in high purity and in good yields.

### BACKGROUND TO THE INVENTION

Extra-chromosomal DNA, such as, for example, plasmid DNA is becoming increasingly important as a biopharmaceutical in its own right. As a consequence, there is a need to generate large amounts of such extra-chromosomal DNA in high purity and in good yields.

Purification schemes that involve the use of large amounts of flammable organic solvents (e.g., ethanol and isopropanol), and toxic chemicals (e.g., ethidium bromide, phenol and chloroform), or techniques such as, ultra centrifugation and "spin columns", whilst adequate for generating small amounts of research material, are not generally suitable for generating the quantities of material needed for biopharmaceutical applications.

In addition many current plasmid purification procedures involve the addition of RNase, typically of bovine origin. Materials derived from bovine sources are increasingly undesirable in the manufacture of pharmaceuticals due to concerns regarding bovine spongiform encephalopathies (BSE's).

In general plasmid purification methods may be considered to comprise two distinct stages:
A first stage which involves releasing the plasmid DNA from their cells to form a lysate; and
A second stage which involves separating and purifying the plasmid DNA from the other cellular components of the lysate.

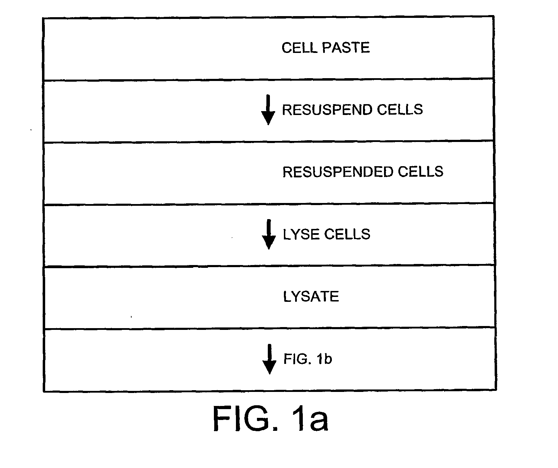

The most common way of implementing the first stage (see figure 1a) is to release plasmid DNA by either:
i) Boiling; or
ii) Using an alkaline pH and detergent mediated solubilization of the bacterial cell membranes.

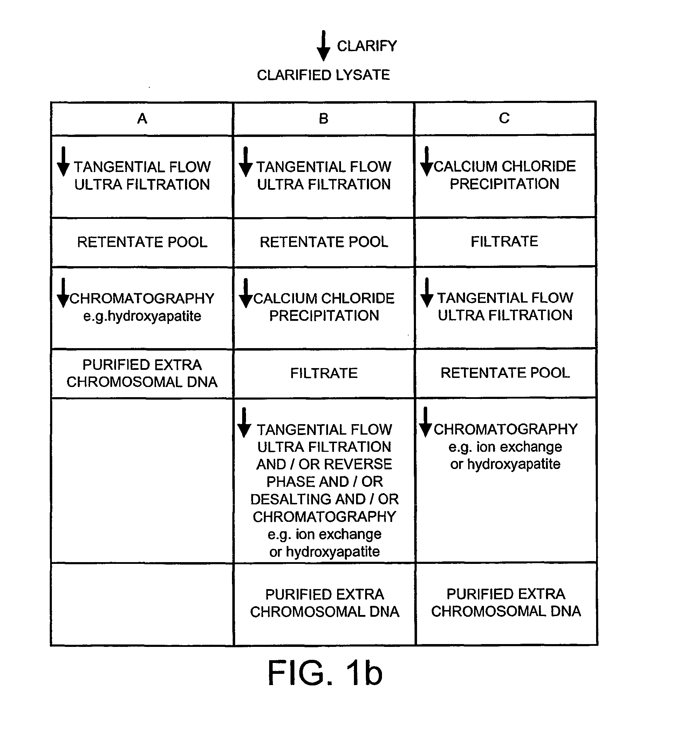

The second stage (see figure 1b) typically involves a clarification step in which insoluble particles, such as, cell debris, are removed followed by a plurality of purification or polishing steps in which, for example, soluble cellular components such as genomic DNA, RNA, proteins and endotoxins are removed. Thus a typical purification may comprise a combination of different purification steps, such as, for example:
Ultra centrifugation through a caesium chloride gradient;
Selective precipitation;
Column chromatography e.g. ion exchange; and
Two phase separation.

One technique used for purifying DNA is tangential flow ultra filtration. PCT/US97/13494 teaches purifying a nucleic acid from a solution comprising filtering the solution through an ultra filtration unit comprising a gel layer to provide a permeate solution and a retentate solution. The nucleic acid is retained in the retentate solution. In order to remove the RNA the lysate is first treated with RNase to digest it to a size which enables separation to be achieved by, for example, ion exchange chromatography.

The process conditions specified include:
1. The use of a Tris-HCl diafiltration buffer with a pH of 8.5 but of unspecified concentration. However since the retentate is loaded onto a chromatography column packed with an anion exchange chromatography resin it can be assumed that the diafiltration buffer has a typical salt concentration of 10-50 mS. This is supported by the comment that where a DNA is to be complexed with a lipid carrier for use in gene therapy it is desirable to exchange the DNA into a low conductivity buffer, preferably by diafiltration. A low conductivity buffer is said to include any buffer of less than 10 mS, preferably less than1 mS;
2. The use of approximately 50 volume exchanges of a diafiltration buffer;
3. The use of an open-channel cassette with a pore size of 300K; and
4. A plasmid loading of 100 mg of nucleic acid/ft² of membrane.

This method suffers from the disadvantage that it requires RNase to digest the RNA in order that the RNA can be separated from the plasmid DNA.

More recently Kahn et al, Biotechnology and Engineering, Vol 60, No 1, July 5 2000 discloses a tangential flow process for plasmid purification that avoids the use of RNase. However in order to remove the RNA they had to first digest it using an extended alkaline lysis lasting 24 hours.

There are two principle disadvantages to this process:
1. Firstly the overall time to run the process is significantly increased and indeed is potentially a rate limiting step; and
2. Secondly the extended lysis runs the risk of damaging the extra-chromosomal DNA itself.

According to the paper the plasmid DNA was prepared by tangential flow under the following conditions:
1. The buffer was a 20 mM Tris-HCl buffer, pH 7.6;
2. The process used 8-10 volume exchanges of diafiltration buffer;
3. The membrane had a pore size of either 500 or 1000 K; and
4. The plasmid loadings were 10-15 g of cells/0.5 ft² of membrane. (Approximately 16 mg of plasmid/ft^{2.})

Other points of note include the fact that the plasmid size varied from between 5.6 Kb and 7.9 Kb for a membrane with a pore size of 500 KDa and was 10.0 Kb for a membrane with a pore size of 1000 KDa.

In both methods it is necessary to first digest the RNA in order that it can be separated from the extra-chromosomal DNA by tangential flow ultra filtration.

It is an aim of the present invention to provide a method for separating DNA from RNA without RNase, which method is simple, readily scalable and does not have the disadvantages associated with the Kahn et al process.

### SUMMARY OF THE INVENTION

The applicant has determined that a substantially RNA free DNA, for example an extra-chromosomal DNA, can be obtained simply and in a readily scalable manner using tangential flow ultra filtration by carefully controlling one or more of the operating conditions.

Whilst the best results are obtained by the careful control of a number of operating conditions it should be recognised that the optimisation of any one or more of the operating conditions may provide significant removal of RNA and consequently each optimised condition is claimed independently as well as in combination with one or more of the other operating conditions.

Thus, the applicant has determined that, contrary to what is suggested by the art, it is possible, by carefully controlling one of more of the operating conditions of the ultra filtration process, to separate extra-chromosomal DNA from RNA, without having to first digest the RNA by either treatment with RNase or by conducting an extended lysis.

These include, but are not limited to, the following:
1. Selecting the buffer on the basis of its ionic strength, which ionic strength can be defined in terms of its conductivity;
2. Providing sufficient volume exchanges of diafiltation buffer to wash the RNA through the membrane;
3. Selecting an appropriate membrane with an appropriate pore size for the extra-chromosomal DNA being purified;
4. Selecting an appropriate trans membrane pressure; and
5. Controlling the loads.

More particularly by optimising both the ionic strength and providing sufficient volume exchanges of the diafiltration buffer, and more preferably still additionally selecting an appropriate membrane with an appropriate pore size for the extra-chromosomal DNA being purified, it is possible to effect substantial RNA removal without the need for either a RNase digest or an extended lysis.

Furthermore, as a consequence of the process the number or complexity of the steps necessary to achieve a desirable level of purification may be reduced. This reduction in the number, or complexity of, purification steps is clearly beneficial since it may reduce processing times, costs and plasmid losses.

According to a first aspect of the present invention there is provided a method of separating extra-chromosomal DNA from RNA without first digesting the RNA, said method comprising:
i) lysing cells comprising extra-chromosomal DNA to form a lysate;
ii) clarifying the lysate; and
iii) subjecting the clarified lysate to tangential flow ultra filtration under conditions which allow a substantial amount of the RNA present in the lysate to pass through a membrane whilst the majority of the extra-chromosomal DNA is retained.

By substantial amount is meant greater than 50%, more preferably greater than 90 %, more preferably still greater than 95%, and most preferably still greater than 98%.

The significant RNA removal achieved with the ultra filtration step means extra-chromosomal DNA of pharmaceutical quality can be obtained with as little as a single further purification or polishing step.

The one or more additional purification or polishing steps may be carried out either before and / or after the ultra filtration step. The step(s) may be to remove additional RNA or other cellular components, such as, for example, endotoxins, proteins, and genomic DNA. It will of course be appreciated that a large proportion of these other cellular components will also be removed during the ultra filtration process. In one embodiment (Figure 1b, route A) the tangential flow ultra filtration step is followed by a chromatography step, such as, for example, hydroxyapatite which step is designed to specifically remove high molecular weight RNA. By high molecular weight RNA is meant RNA with a molecular weight approaching that of the extra-chromosomal DNA from which it is being separated

In yet another embodiment (Figure 1b, route B) an additional RNA removal step is conducted following the ultra filtration step. Preferably this is a CaCl₂ precipitation step designed to specifically remove high molecular weight RNA.

The CaCl₂ precipitation step may be followed by a further ultra filtration step and / or a desalting step and / or a reverse phase chromatography step, which in turn may be followed by a chromatography step, such as, for example ion exchange or hydroxyapatite.

In yet a further embodiment (Figure 1b, route C) a RNA removal step.e.g. a CaCl₂ precipitation step, precedes the ultra filtration step. In this embodiment the ultrafiltration step which succeeds the CaCl₂ precipitation step could be followed by a chromatography step, such as, for example, an ion exchange or a hydroxyapatite step.

The use of an antichaotropic salt, such as, for example, ammonium sulphate, sodium sulphate, potassium citrate, calcium chloride, ammonium acetate or potassium acetate, but particularly calcium chloride can effect the choice of membrane for ultrafiltration.

The conditions giving rise to substantial removal of RNA include the following:
1. The selection of a buffer having a low ionic strength. This selection proved critical in maximising RNA removal. The lower the ionic strength the greater the removal of RNA. By low ionic strength is generally meant having a conductivity of less than 10 mS, more preferably less than 5 mS, more preferably still less than 2 mS and most preferably less than 1 mS. The preferred buffer is a Tris-HCl buffer or a Sodium phosphate buffer although other buffers which work in the pH range 6 - 10 may be suitable. Examples of other such buffers include: Sodium carbonate / CO₂; Imidazole; 2,4,6-trimethylpyridine; MOPS; Sodium diethyl barbiturate; N- ethylmorpholine; HEPES; TRICINE; EPPS; 2-amino-2-methyl-1, 3-propanediol; BICINE; Sodium borate and Glycine. The preferred pH is between about 7.5 and 8.5.
2.The use of a sufficient number of volume exchanges during diafiltration to ensure the RNA is washed through the membrane. Preferably there are at least 20 volume exchanges, more preferably at least 30 volume exchanges, more preferably still at least 40 volume exchanges and most preferably 50 or more volume exchanges, for example between 50 and 100.
3.The selection of an appropriate membrane. Preferably a polyethersulphone membrane with a pore size of from 300 KDa to 500 KDa is selected although other suitable membranes, including cellulose acetate, polysulphone, polyvinylidene, polypropylene, polyamide, modified PES, polyvinylidine pyrrolidine, polyvinylidine fluoride (PVDF), cellulose nitrate and cellulose triacetate may be used.
   The selection of the pore size is important since if too large a pore size is selected plasmid will be lost through the membrane and if too small a pore size is selected the RNA will not be able to pass through the membrane. Thus, the pore size could infact range from 50 KDa to 1000KDa, more typically 100 KDa to 1000 KDa depending upon whether the extra chromosomal DNA was particularly large or small. Thus the actual selection will depend upon the plasmid size and operating conditions.
   Suprisingly, when calcium chloride is used it has been found necessary to use a membrane with a smaller pore size than would normally be predicted for a plasmid of a given size.
   Thus, a membrane with a pore size of 100K has proved to be particularly effective for plasmids of 5.9kbp and 7.7kbp.
4. The starting trans membrane pressure (TMP) has also been found to be significant in achieving good plasmid yields. Preferably the starting TMP is less than 10 psi, more preferably still less than 8 psi, more preferably still less than 6 psi, and most preferably about 5 psi. It is preferred to maintain this at or below 15 psi most preferably at or below 10 psi.
5. Plasmid loads have also been found to have a significant effect on yield and it is preferred if the loads are at the lower ends of the following ranges:
   For a 300K membranes, total nucleic acids loadings ranged from 200-1000mg/ft² (10-50mg/ft² plasmid). Thus for a 300K membrane the preferred loadings are less than 30mg/ft² plasmid, more preferably less than 20 mg/ft² plasmid.
   For a 500K membrane, total nucleic acid loadings ranged from 1000-3000mg/ft² (50-150mg/ft² plasmid). Thus for a 500K membrane the preferred loadings are less than 100mg/ft² plasmid, preferably less than 75mg/ft² plasmid
   Loading levels appeared less significant on the 100K membrane in the presence of calcium chloride.

In the experiments giving rise to the observations 1 to 3 above a TMP was generally maintained at between 10 and 20 psi and a cross flow rate was generally maintained at between 0.5 to 1.0 L/min/ft². The combination gave rise to a permeate flux rate of between 16 and 26 L/h/m². As noted in 4 above a lower TMP has since been found to improve plasmid yields.

Whilst it was thought necessary to polarise the membrane (by recirculating the permeate line for a period sufficient to form a gel layer) to maximize plasmid yields this has unexpectedly been found not to be necessary, in a RNase free process, particularly for a first stage ultrafiltration step UF1.

According to a second aspect of the present invention there is provided a method of generating extra-chromosomal DNA in high purity and in good yields in a tangential flow ultra filtration process which method comprises utilising a screen designed to create turbulence in a retentate channel of an ultra filtration apparatus.

Whilst CaCl₂ precipitation of RNA is a simple and effective way of removing RNA and genomic DNA contaminants from plasmid solutions, the presence of CaCl₂ results in plasmid losses when the plasmid solution is concentrated or diafiltered by tangential flow ultra filtration.

It is thought that CaCl₂ compacts the plasmid resulting in losses through the ultra filtration membrane.

By removing the CaCl₂ (and also RNA) using a suitable matrix and ion pairing agent, a subsequent reverse phase chromatography step was found to provide particularly effective separation (good purity and high plasmid yields).

According to a third aspect of the present invention there is provided a method of generating extra-chromosomal DNA of high purity in good yields in a purification process comprising a CaCl₂ precipitation step, characterised in that a reverse phase chromatography step follows the CaCl₂ precipitation step.

By employing a reverse phase column chromatography step after the CaCl₂ precipitation step the process may be reduced to a single post CaCl₂ step.

According to a fourth aspect of the present invention there is provided a method of purifying DNA from other cellular components characterised in that the DNA is separated using hydroxyapatite chromatography at a pH of from 7.3 to 8.3.

According to a further aspect of the present invention there is provided a purified extra-chromosomal DNA; a pharmaceutical composition, a DNA vaccine or gene therapy product obtained by a method of any of the inventions claimed.

According to yet a further aspect of the present invention there is provided a purified extra-chromosomal DNA comprising:
Less than 0.13 % by weight protein when tested using a BCA kit;
Less than 0.11 % by weight genomic DNA when tested using a Q-PCR method
Less than 5 EU/mg endotoxin when tested using a LAL test; and
Less than 1.0 % by weight residual RNA when tested using a HPLC assay

The inventions will now be described, by way of example only, with reference to the following detailed description and examples:

### BRIEF DESCRIPTION OF THE DRAWINGS

Figure 1a is a schematic of the processing steps involved in obtaining a lysate;
Figure 1b is a schematic showing three alternative routes of obtaining pharmaceutical grade extra-chromosomal DNA using a process according to the invention;
Figure 2a is an ion exchange HPLC trace of the lysate of example 1;
Figure 2b is an ion exchange HPLC trace of the diafiltration permeate of example 1;
Figure 2c is an ion exchange HPLC trace of the retentate pool of example 1;
Figure 3a is an ion exchange HPLC trace of the lysate to be diafiltered against the 500 mM buffer, pH 7.5, of example 2;
Figure 3b is an ion exchange HPLC trace of the retentate pool obtained with the 500 mM buffer, pH 7.5, of example 2;
Figure 3c is an ion exchange HPLC trace of the lysate to be diafiltered against the 100 mM buffer, pH 7.5, of example 2;
Figure 3d is an ion exchange HPLC trace of the retentate pool of the 100 mM buffer, pH 7.5, of example 2;
Figure 3e is an ion exchange HPLC trace of the lysate to be diafiltered against the 10 mM buffer, pH 7.5, of example 2;
Figure 3f is an ion exchange HPLC trace of the retentate pool of the 10 mM buffer, pH 7.5, of example 2;
Figure 4a is an ion exchange HPLC trace of the lysate to be diafiltered against the 10 mM buffer, pH 6.0, of example 3;
Figure 4b is an ion exchange HPLC trace of the retentate pool of the 10 mM buffer, pH 6.0, of example 3;
Figure 4c is an ion exchange HPLC trace of the lysate to be diafiltered against the 10 mM buffer, pH 9.0, of example 3;
Figure 4d is an ion exchange HPLC trace of the retentate pool of the 10 mM buffer, pH 9.0, of example 3;
Figure 5 is a plot of permeate flux versus diafiltration volumes for the buffers of example 4;
Figure 6 is a plot of TMP verses diafiltration volumes for the buffers of example 4;
Figure 7a is an ion exchange HPLC trace of the lysate of example 5;
Figure 7b is an ion exchange HPLC trace of the retentate pool of example 5;
Figure 8a is an ion exchange HPLC trace of the lysate of example 6;
Figure 8b is an ion exchange HPLC trace of the concentration permeate of example 6;
Figure 8c is an ion exchange HPLC trace of the diafiltration permeate (10 volume equivalents) of example 6;
Figure 8d is an ion exchange HPLC trace of the diafiltration permeate (20 volume equivalents) of example 6;
Figure 8e is an ion exchange HPLC trace of the diafiltration permeate (30 volume equivalents) of example 6;
Figure 8f is an ion exchange HPLC trace of the diafiltration permeate (40 volume equivalents) of example 6;
Figure 8g is an ion exchange HPLC trace of the diafiltration permeate (50 volume equivalents) of example 6;
Figure 8h is an ion exchange HPLC trace of the retentate pool of example 6;
Figure 8i is an ion exchange HPLC trace of the retentate pool of example 6 at twice the loading compared to fig 8h;
Figure 9a is an ion exchange HPLC trace of the permeate of example 8 after 20 minutes recirculation;
Figure 9b is an ion exchange HPLC trace of the lysate of example 8;
Figure 9c is an ion exchange HPLC trace of the retentate of example 8 after 20 minutes recirculation;
Figure 9d is an ion exchange HPLC trace of the permeate during concentration of example 8;
Figure 9e is an ion exchange HPLC trace of the permeate during diafiltration, first 150 ml, of example 8;
Figure 9f is an ion exchange HPLC trace of the permeate during diafiltration, second 150 ml, of example 8;
Figure 9g is an ion exchange HPLC trace of the permeate during diafiltration, third 150 ml, of example 8;
Figure 9h is an ion exchange HPLC trace of the permeate during diafiltration, fourth 150 ml, of example 8;
Figure 9i is an ion exchange HPLC trace of the retentate pool of example 8 after concentration and diafiltration;
Figure 9j is an ion exchange HPLC trace of the wash 1 of example 8;
Figure 9k is an ion exchange HPLC trace of the wash 2 of example 8;
Figure 10a is an elution profile showing plasmid DNA separation with 10-500 mM Na₂HPO₄ at pH 6.8;
Figure 10b is an elution profile showing plasmid DNA separation with 10 -500 mM Na₂HPO₄ at pH 7.6;
Figure 10c is an elution profile showing plasmid DNA separation with 10 -500 mM Na₂HPO₄ at pH 7.8; and
Figure 10d is an elution profile showing plasmid DNA separation with 10 -500 mM Na₂HPO₄ at pH 8.8.

### DETAILED DESCRIPTION OF THE PREFERRED EMBODIMENTS

Having unexpectedly found that RNA could be separated from DNA in a tangential flow ultra filtration process without first conducting a RNA digest a basic protocol was devised and one or more factors modified in an attempt to determine which factors had a significant effect on the removal of cellular components, particularly RNA.

A schematic illustrating a number of alternative separation routes (1A, 1B and 1C) is set out in Figs 1a and 1b and fuller processing details are described in more detail below:

### RNase-free plasmid purification protocol: 300g scale

### 1. Cell resuspension

2.5L of resuspension buffer (25 mM Tris/10 mM EDTA/55 mM Dextrose pH 8.0.) was added to 300 g of cell paste to resuspend the cells.

### 2. Alkaline lysis

Lysis buffer (4.17L 0.96% NaOH, 830ml 6% SDS) was added to the cell suspension and mixed until homogenised thus forming a lysate.

### 3. Potassium acetate precipitation

2.5L of 3M potassium acetate was added to the lysate, stirred for 25-35 minutes and centrifuged at 4600RPM for 20mins. The supernatant was decanted off through a double layer of Miracloth. The cell pellet was weighed and discarded. The supernatant was passed through 1 x 1.2µm Sartopure PP and 1 x 0.45/0.2µm Sartobran P filters. The filtrate is the clarified lysate.

### 4.Ultrafiltration

### Materials:

Millipore rig with 3 x 5sq ft 300K PES, Omega Centrasette, open channel membranes.
This is stored in 0.1M NaOH between runs.
Millipore Masterflex peristaltic pump.
Diafiltration buffer: 20mM Tris pH 7.5.

### Prepare system

Empty contents of reservoir and membrane cassettes through the permeate line.
Fill the reservoir with 2L of diafiltration buffer and recirculate through the retentate line with the permeate line closed.
Empty contents of reservoir and membrane cassettes through the permeate line.
Fill the reservoir with 2L of diafiltration buffer, connect the buffer bottle to the reservoir and flush 5L of diafiltration buffer through the permeate line. Check the pH of the permeate.

### Formation of Gel Layer:

Empty system.
Fill reservoir with approx 1.8L clarified lysate and connect butt containing lysate to system.
Recirculate at a starting pressure of 10 psi, with permeate line going back into the lysate vessel, for 30minutes (to build up gel layer).
Keep an eye on the retentate pressure as it may rise quickly. It should not exceed 20 psi. Adjust back down to 20 psi if this occurs.

### Concentration

Concentrate lysate to 500ml. Collect concentration permeate.

### Diafiltration

Diafilter at 20 psi using approximately 50 volumes of diafiltration buffer (25L). Use continuous diafiltration mode where filtrate is replaced by fresh buffer at the same rate. Collect diafiltration permeate in 5x5L bottles. Record volume, pH and conductivity.

### Harvest

At the end of diafiltration, recirculate contents of reservoir and cassettes for 30 minutes with the retentate valve open and the permeate line closed. Drain the UF rig into a 500ml bottle (UF1-D permeate).

Add 500ml of fresh diafiltration buffer to the reservoir and recirculate for 30 minutes. Drain the rig to a 500ml bottle (UF1-D permeate wash 1).

Repeat above (UF1-D permeate wash 2).

Pool UF1-D permeate, permeate wash 1 and permeate wash 2.

Filter retentate pool through 1 x Gelman Science 0.45 µm filter (and if required 1 x N66 Ultipor 0.2 um).

The effect of varying one or more of the operating conditions from this protocol is set out with reference to examples 1 to 10 below whilst examples 11 to 15 show further variations:

### EXAMPLE 1 (Effect of ionic strength part 1)

A very significant finding occurred when a 10mM Tris + 0.45M NaCl diafiltration buffer, pH 8.5 (conductivity 41.3 mS) was replaced with a 0.1 M Tris diafiltration buffer, pH 8.5 (conductivity 6.89 mS).

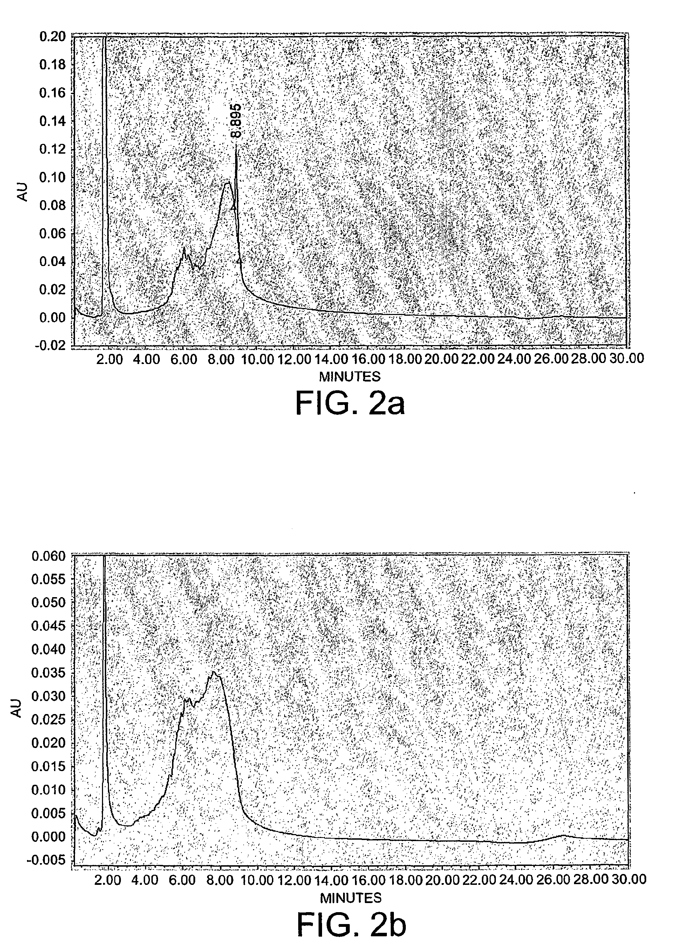

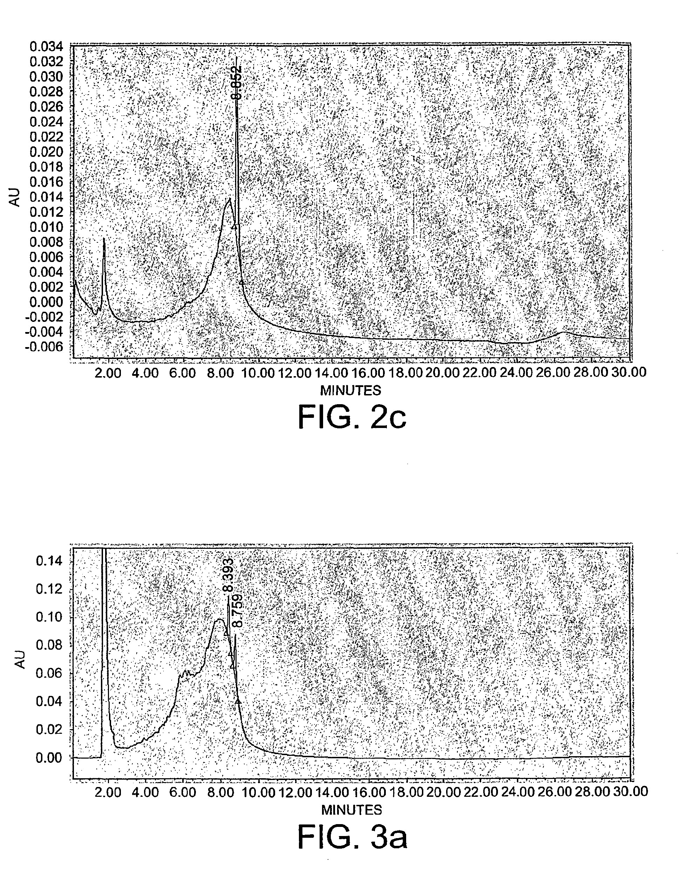

When this low ionic strength buffer was used clearance of a significant amount of RNA was observed. This was evidenced by ion exchange HPLC where a diafiltration permeate sample showed the presence of RNA but no plasmid (Fig 2b) whereas the lysate showed the presence of both RNA and plasmid (Fig 2a). The retentate pool also showed removal of RNA as compared to the lysate. (Fig2 c)

The above results were confirmed and quantified by conducting a RNA assay by reversed phase HPLC. The results indicated that:
32% of RNA found in the lysate was present in the diafiltration permeate;
43.7% of the RNA found in the lysate was present in the retentate pool; and
0.9% of the RNA was found in the concentration permeate.

Whilst the applicant does not wish to be bound by theory the results suggest that low molecular weight RNA originally participates in the gel layer but is then allowed to resolubilise by the diafiltration buffer and is able to pass through the ultrafiltration membrane.

### EXAMPLE 2 (Effect of ionic strength part 2)

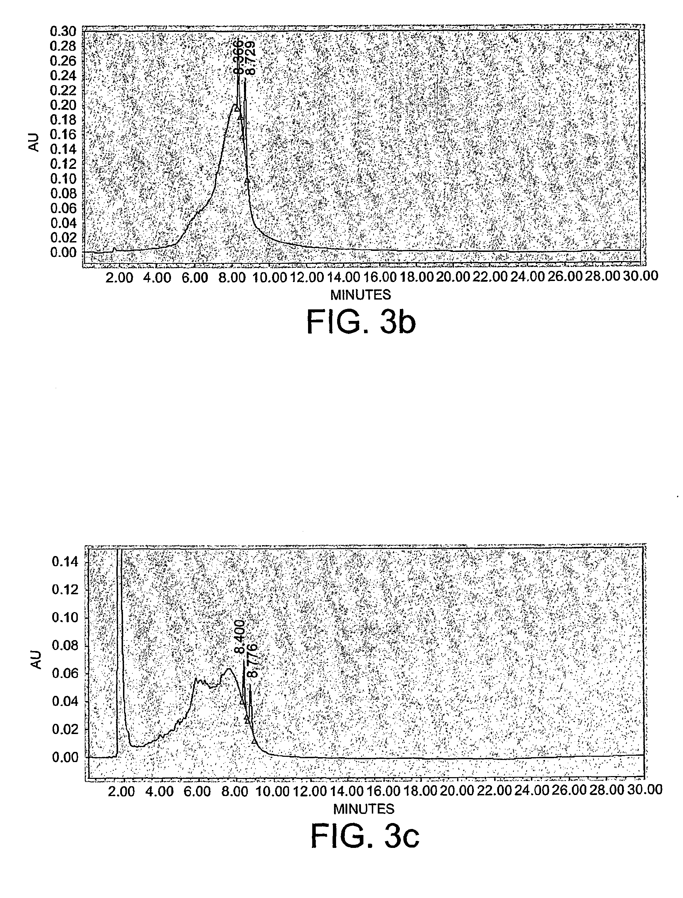

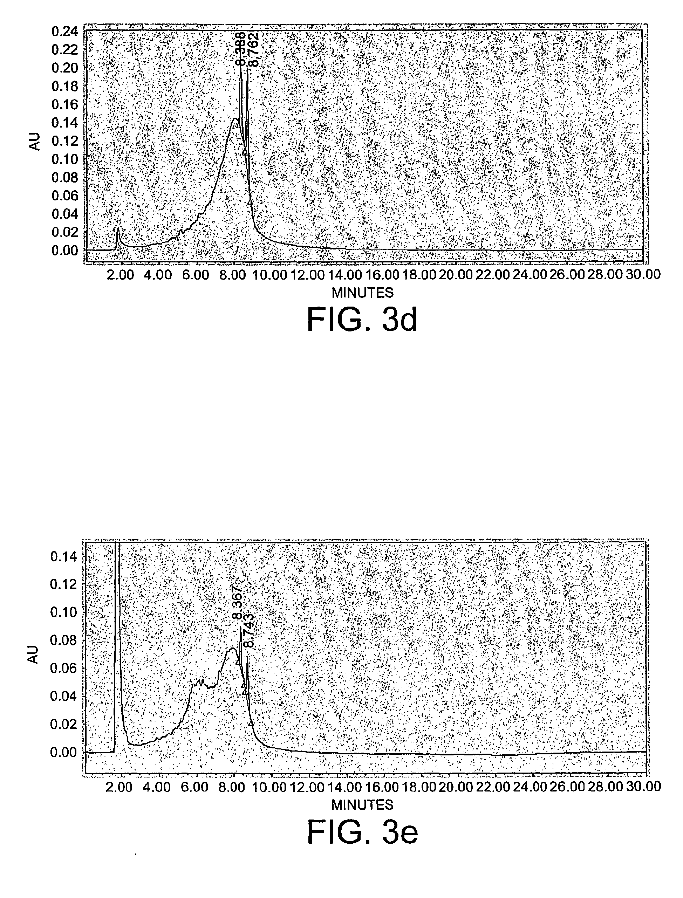

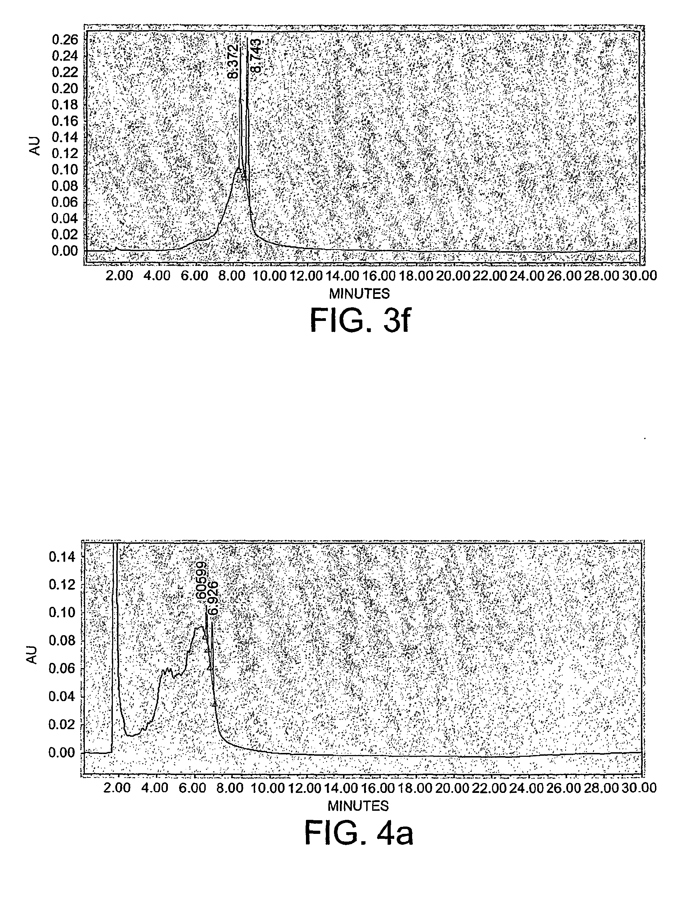

A further experiment was conducted to determine the significance of the ionic strength on RNA removal. Three Tris buffers of different ionic strengths were compared. The Tris buffers all had a pH of 7.5, but were of varying concentrations as set out below:
500 mM (conductivity 25.3)
100 mM (conductivity 6.89)
10 mM (conductivity 0.33)
A comparison of the HPLC traces (lysate versus retentate pool) demonstrated that the lower the ionic strength the greater the RNA removal (Figs 3 a to 3f).

### EXAMPLE 3 (Effect of pH)

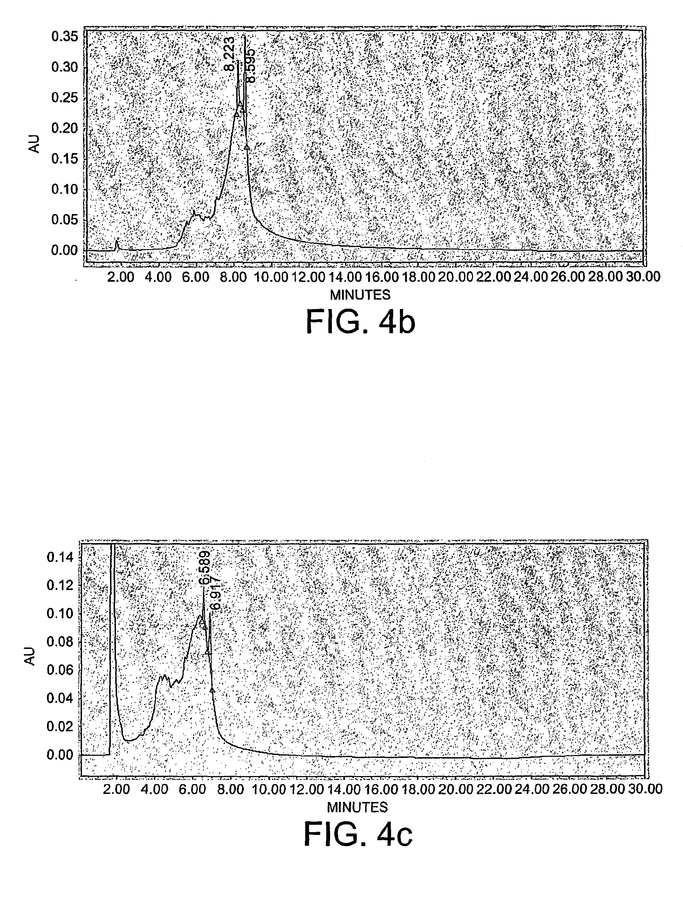

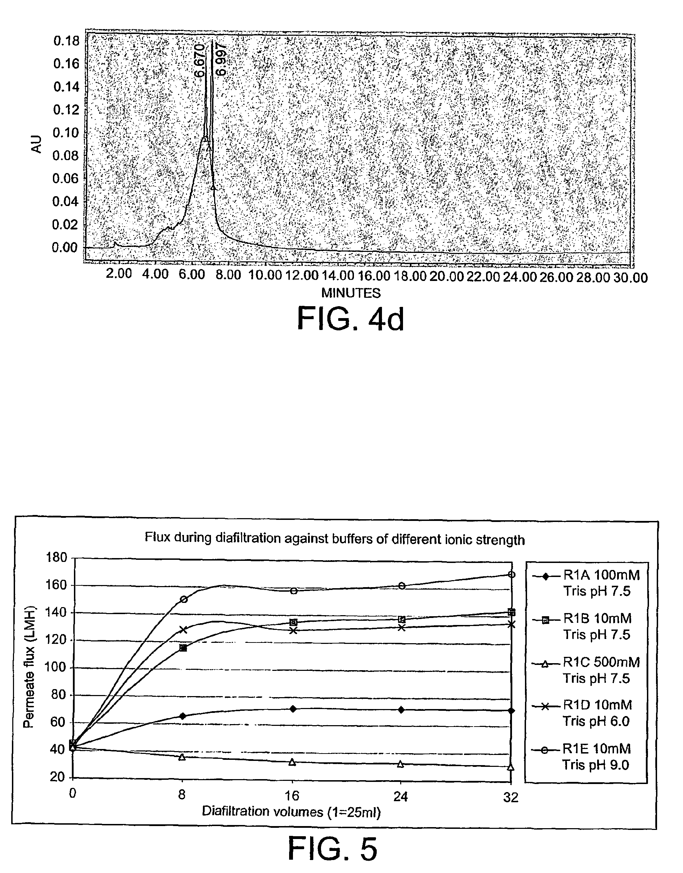

Having deduced that ionic strength affected RNA removal the effect of pH on RNA removal was investigated. The investigation was made against a 10 mM Tris buffer at a pH of 6, 7.5 and 9. Again by comparing HPLC traces (lysate versus retentate pool) it was determined that a pH between 7.5 and 9 gave better RNA clearance than a pH of 6 although the results at pH 9 were no better than at pH 7.5 (Fig 4a to 4d and Fig 3e and 3f.)

### EXAMPLE 4 (Effect of ionic strength on permeate flux and trans membrane pressure)

As well as affecting RNA clearance it was noted that the permeate flux and trans membrane pressure (TMP) was affected by the ionic concentration of the diafiltration buffer. Thus whilst the permeate flux was generally in the order of 40 L/m²/h at the commencement of diafiltration it altered with increasing volume exchanges. In the case of the 500mM buffer the permeate flux dropped to about 30 L/m²/h whilst for the 100 mM buffer it increased to about 70 L/m²/h and for the 10 mM buffer it increased to about 140 L/m²/h. These finding are significant in that they demonstrate that processing can be conducted at faster speeds for the lower ionic strength buffers; by as much as a factor of 5. The results are shown graphically in Fig 5.

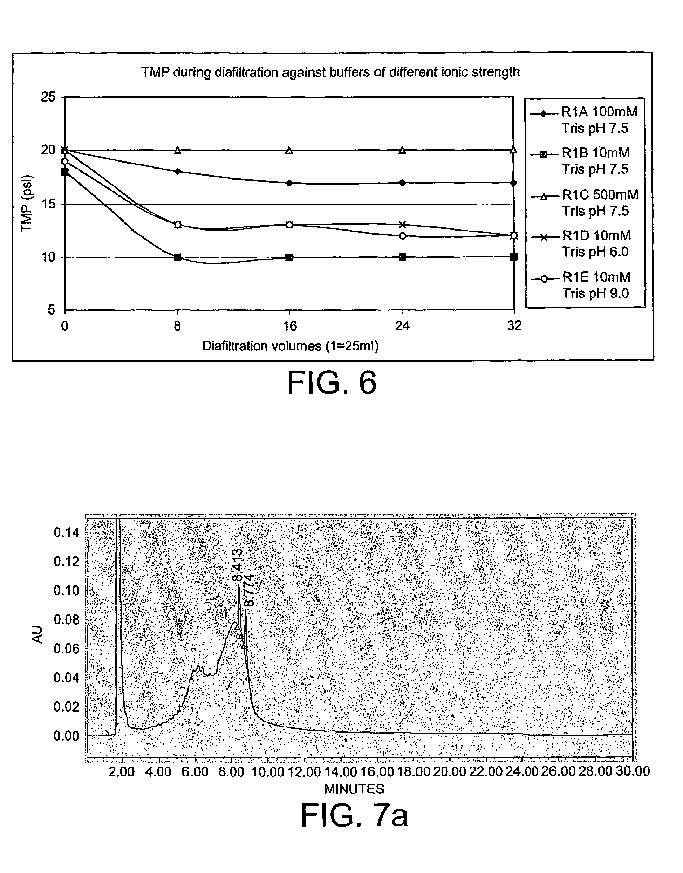

Also the TMP dropped from a starting pressure of about 20 to about 17 in the case of the 100 mM buffer and to about 10 in the case of the 10 mM buffer. This is in contrast to the 500 mM buffer for which the TMP remained constant. The results are illustrated in Fig 6.

### EXAMPLE 5 (Effect of volume exchanges)

Surprisingly, it was found that in the case of the 10 mM buffer significant quantities of RNA were still passing through the membrane after 32 volume exchanges. Consequently a further experiment was conducted to determine when RNA stopped being eluted.

500 ml of clarified lysate was removed from -20 °C storage, thawed and filtered through a 0.22 µm filter. 150 ml of filtered lysate was placed in the reservoir of a TFF rig and pumped around the system at a cross flow rate of 550 ml/min/ft². The TMP was set at 0.6 Bar by partially closing the retentate valve of the TFF rig. The lysate was recirculated for 20 minutes to polarise the membrane. The contents of the reservoir were concentrated to approximately 25 ml and then diafiltered against 1600ml of diafiltration buffer.

At the end of diafiltration, the permeate line was closed and the retentate valve opened to eliminate TMP. The retentate was then recirculated for 10 minutes to wash the membrane and then harvested to a clean Nalgene bottle. A further 30 ml of diafiltration buffer was pumped around the system for 10 minutes to wash the membrane and then harvested. A total of two washes were performed.
TMP and permeate flux were measured throughout the process, and samples were taken for ion exchange HPLC as an indication of RNA clearance.

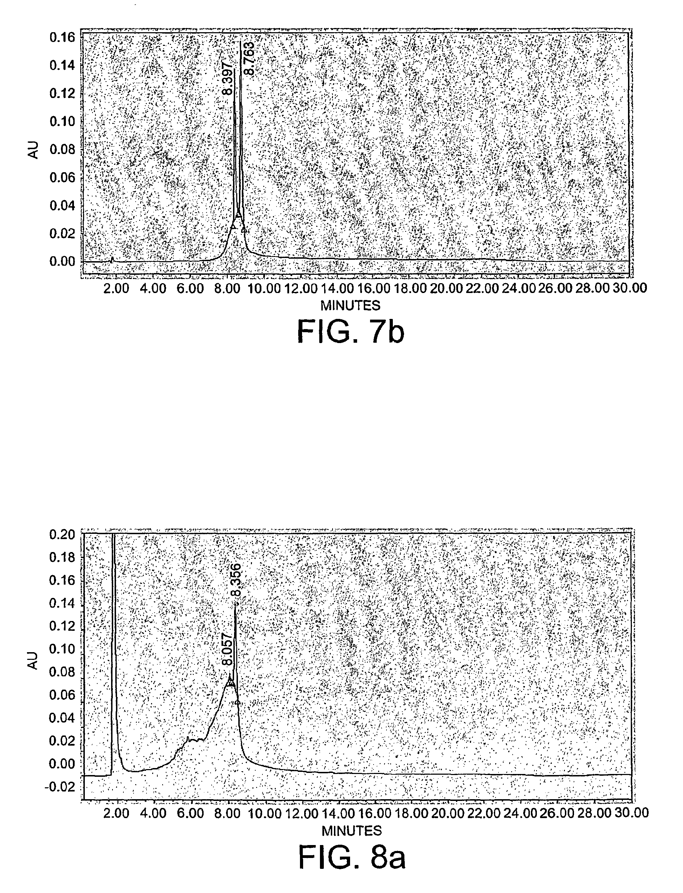

It was found that with an extended diafiltration all but 2% of the RNA present in the lysate could be removed (Fig 7a and 7b). Furthermore, it will be apparent from Fig 7b that the remaining RNA peak is immediately under the plasmid peak indicating that the remaining RNA is all high molecular weight RNA. This data suggests that the process could be used in tandem with a process that is effective at removing high molecular weight RNA, such as, for example CaCl₂ precipitation to ensure the removal of all RNA from a sample.

### EXAMPLE 6 (Effect of plasmid load)

The applicant unexpectedly determined that by reducing the amount of plasmid loaded onto a membrane one could further increase separation of RNA from plasmid DNA. Indeed on reducing plasmid loads from 22.2 mg of plasmid/ft² to 11.1 mg/ft² on a 300 KDa membrane they were able to obtain plasmid DNA with significantly lower levels of RNA present.

The results are shown in Figs 8a to 8i.

To explain, a 7.6Kbp plasmid was diafiltered against 50 volume equivalents of 20mM Tris pH 7.5 on a 300K membrane as per the general protocol. The IE HPLC chromatographs for the sample loaded at 11.1 mg of plasmid per ft² of membrane are shown in Figs 8a to 8h.
Fig 8a is the lysate;

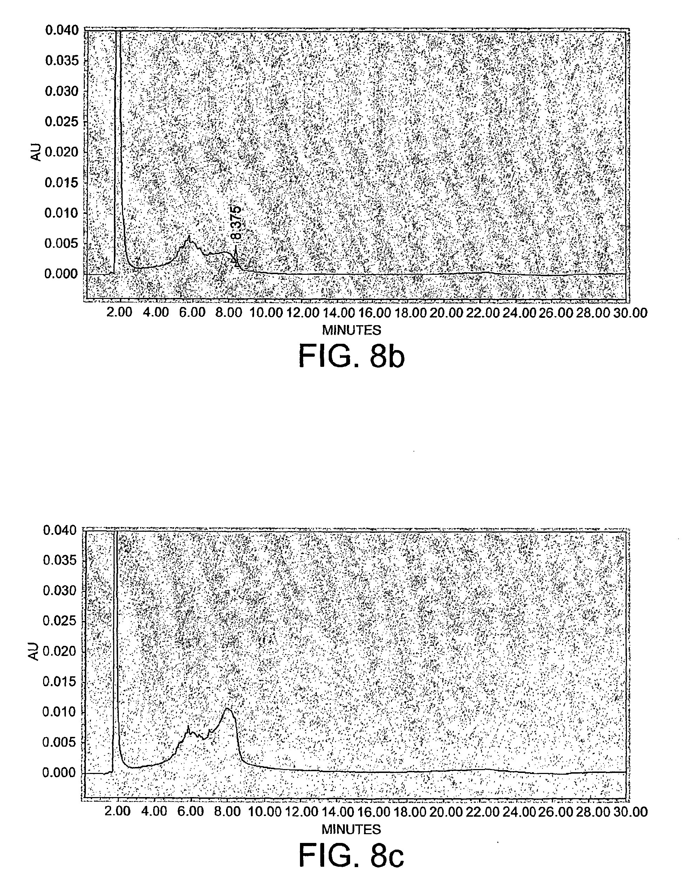

Fig 8b is the concentration permeate;
Fig 8c is the pooled diafiltration permeate volumes 1-10;

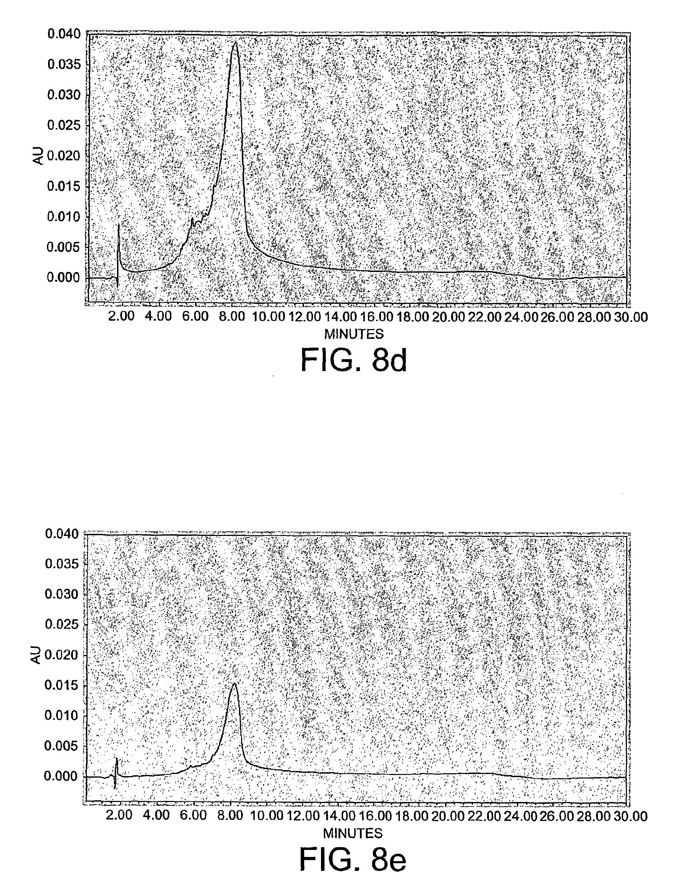

Fig 8d is the pooled diafiltration permeate volumes 11 -20;
Fig 8e is the pooled diafiltration permeate volumes 21-30;

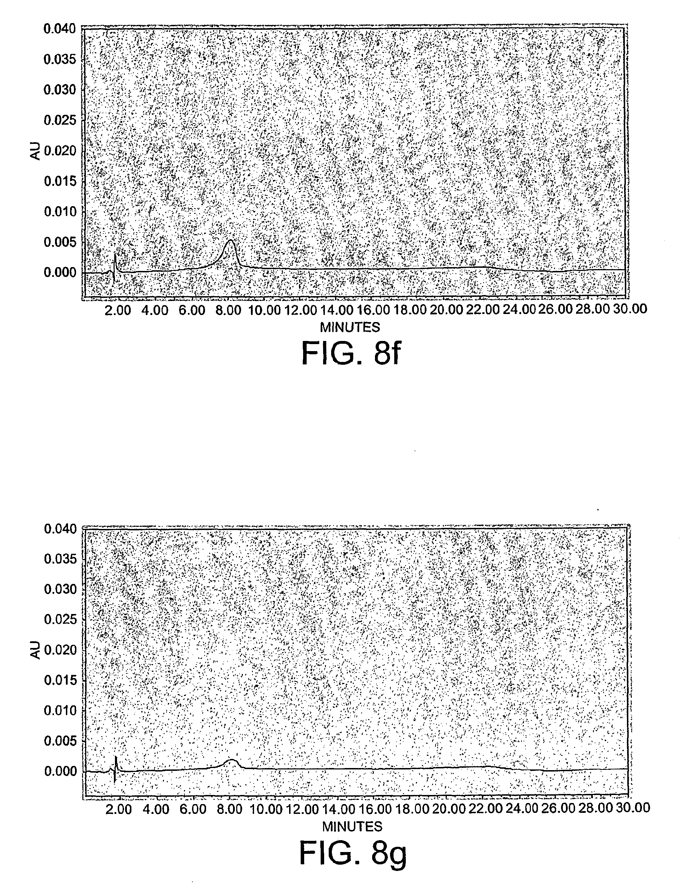

Fig 8f is the pooled diafiltration permeate volumes 31-40;
Fig 8g is the pooled diafiltration permeate volumes 41-50; and

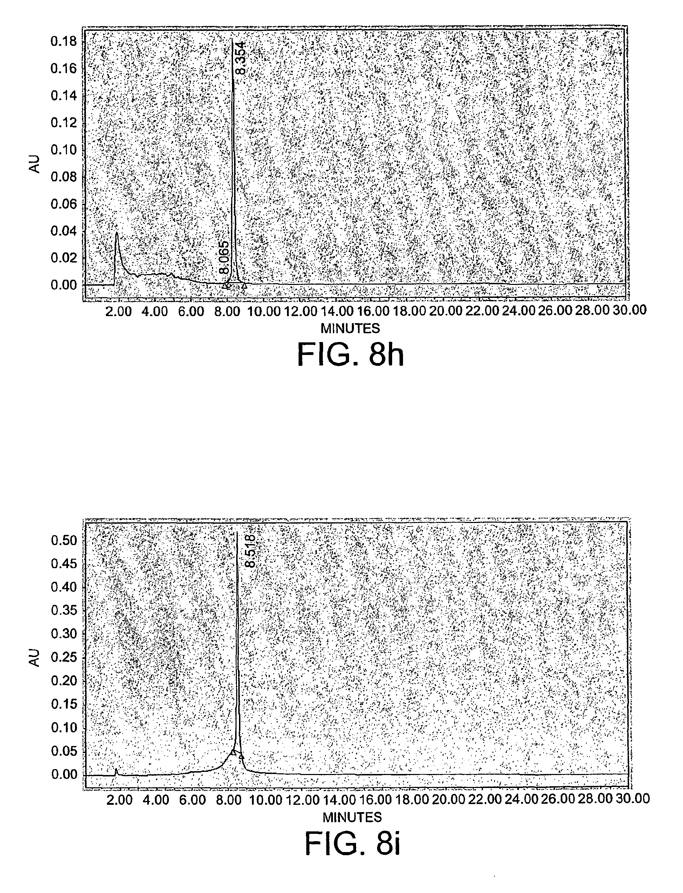

Fig 8h is the retentate pool.
By comparison Fig 8i is the retentate pool of the 22.2 mg of plasmid per ft² of membrane sample.
What the results demonstrate is that at the lower loadings the plasmid is free of RNA. (Compare Fig 8h against Fig 8i.)

The IE HPLC chromatographs also demonstrate how RNA is cleared with increased diafiltration volume equivalents. (Compare 8c to 8 g and also 8a and 8b)
Thus in the lysate (Fig 8a) one can see a first peak to the left (protein) followed by a broad RNA peaks with the plamid peaks 8.057 and 8.356 also marked. The two plasmid peaks are the open and super coiled DNA peaks respectively.
In the concentration permeate (Fig 8b) one can see the protein peak, but very little RNA suggesting that the RNA initially participates in the gel layer. As increasing volumes of the diafiltration buffer are added there is a marked change in the amount of RNA being eluted. After 10 volumes (Fig 8c), only low levels of RNA are cleared through the membrane; after 20 volumes, when the diafiltration buffer is really taking effect, as evidenced by a drop in pH and conductivity, a peak of RNA is cleared through the membrane (Fig 8d). From 30 to 50 volumes the RNA levels can be seen to tail off until by the time 50 volumes have passed through the membrane the levels are not significant and filtration can be stopped. The retentate pool (Fig 8h) shows pure plasmid and no RNA.

The applicant has unexpectedly determined that when using membranes with a larger pore size the plasmid loadings can be higher. For example, with a 500 KDa membrane loadings as high as 150 mg of plasmid/ft² may be used. Indeed the total area of the membrane may in itself be significant since early indications suggest the larger the membrane area the greater the plasmid loadings that can be used.

### EXAMPLE 7 (Additional polishing step)

Whilst the use of a low ionic strength buffer in combination with sufficient volume exchanges of the buffer was capable of removing 98.6% of the RNA present immediately after lysis (10mM, Tris pH 7.5 and 60 volume exchanges) the applicant sought to determine whether a second and different technique could remove any remaining RNA particularly, high molecular weight RNA, to levels whereby the RNA proved undetectable.

They undertook a chromatography-based procedure following the ultrafiltration step. The chromatography step tested was a. hydroxyapatite step. Thus the retentate pool was loaded onto a hydroxyapatite column (Bio-Rad MacroPrep Ceramic Type II, 20µm bead size) under conditions that prevent binding of RNA (0.3 M Na₂HPO₄ pH 7.8) so that the RNA is cleared in the flow-through while plasmid binding is maximised. Plasmid was then eluted with a recovery of 93.4 %. No RNA was detected by ion exchange HPLC.

Interestingly, the applicant also determined that pH proved critical to the effective use of hydroxyapatite to separate RNA from the plasmid DNA. The results demonstrating the effect of pH on the separation of RNA from plasmid DNA are shown in Example 10 which also shows the benefits of using a double buffer system in the separation process.

### EXAMPLE 8 (Effect of channel)

It is generally accepted that an open channel more readily allows the formation of a gel layer which prevents plasmid DNA passing through the membrane and into the permeate. Indeed, it has even been suggested that the use of an open channel cassette may be critical to the successful operation of the process.

Because the applicant experienced difficulties recovering the plasmid from the membrane at the end of the ultrafiltration step, but found badly fouled membranes could be restored by means of a nucleic acid digest, they surmised that the nucleic acid associated with the gel layer was in fact binding irreversibly to the membrane. In an effort to determine if they might disrupt this binding they used a screen designed to cause turbulence in the retentate channel. The protocol was otherwise as described below and a T-screened channel "Omega" membrane was used.

180 ml of clarified lysate (RNase treated) was placed in the reservoir of a Minim system. The pump was switched on to give a cross flow rate of 100ml/ min. The retentate valve was closed slightly to give a TMP of 0.8 Bar (inlet pressure 1.5 Bar, outlet pressure 0.1 Bar.). The system was run in total recirculation mode for 20 minutes to allow a gel layer to form. Samples from the permeate and reservoir were taken for HPLC analysis. The permeate line was placed in a clean bottle, and the lysate was concentrated to a final volume of 30 ml. The concentrate was then diafiltered against 20 volumes (600 ml) 10 mM Tris / 0.45 M NaCl pH 8.5. Four 150 ml permeate samples were collected during diafiltration and one during concentration and analysed by HPLC. The retentate valve was opened and the permeate line clamped to remove TMP. The system was left for 10 minutes to dissolve the gel layer and harvest the plasmid. After 10 minutes, the retentate was harvested from the reservoir and replaced with 30 ml diafiltration buffer. This was recirculated for 10 minutes as before, harvested into a separate container, and labelled "wash 1". The wash step was repeated .The volumes of retentate harvest, wash 1 and wash 2 were measured and the samples taken for HPLC analysis. The absorbance at 260 nm and 280 nm was also measured and the 260/280 ratios calculated.

Two criteria were used to measure the effect of using a screened channel. These were, the recovery of plasmid from the process, and the clearance of contaminants by visual inspection of HPLC chromatograms.

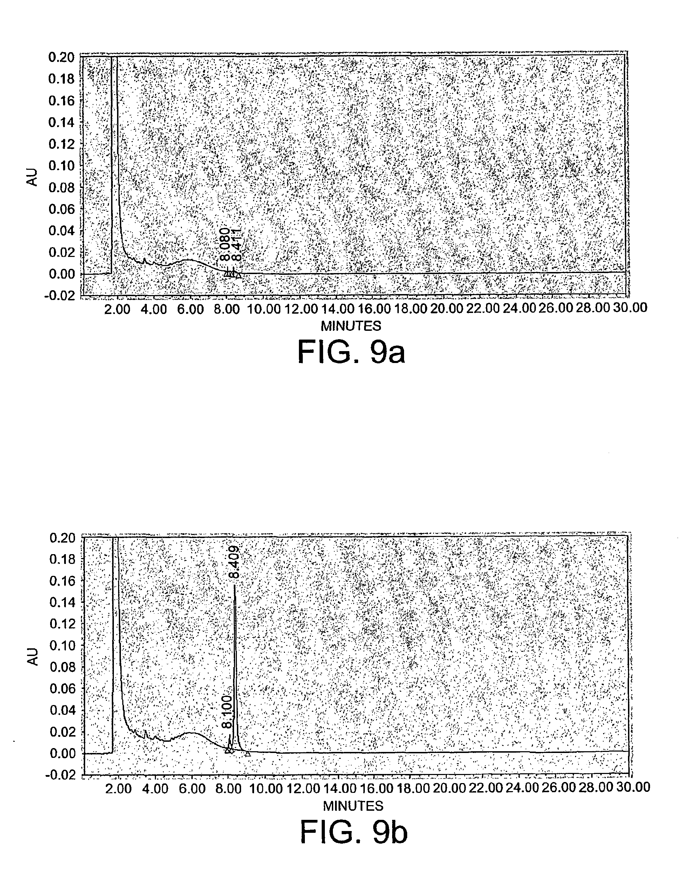

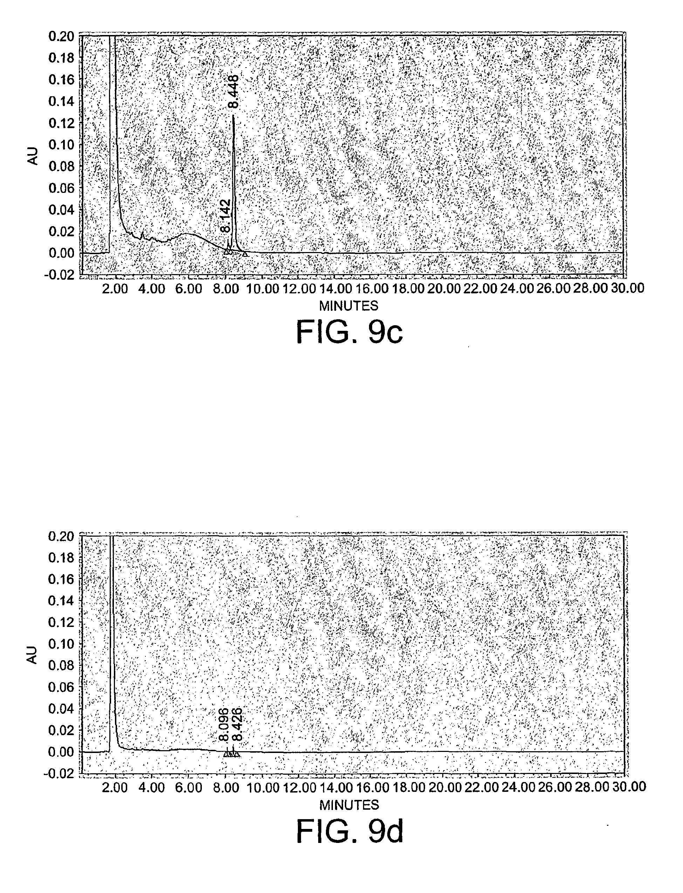

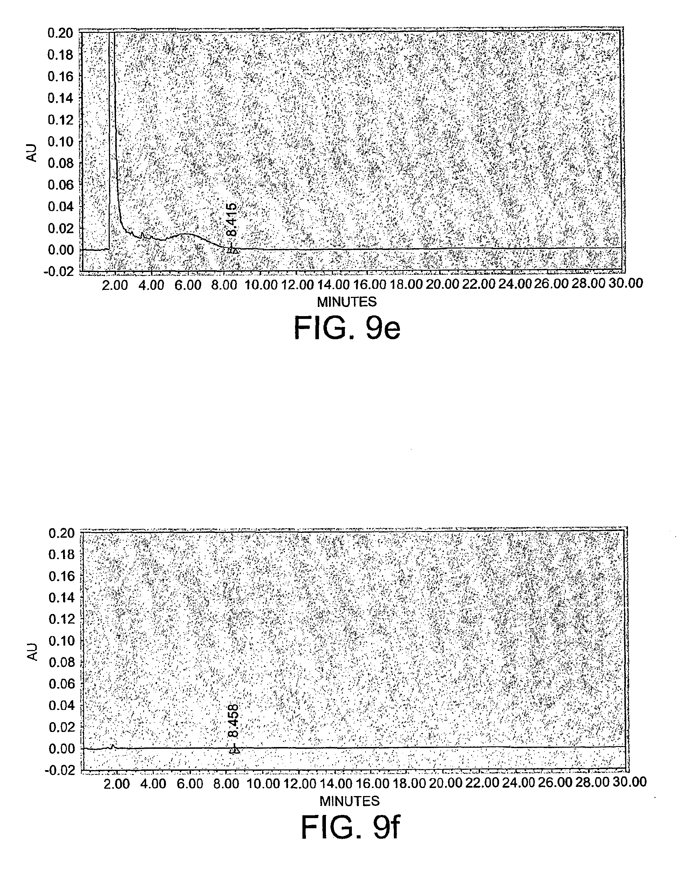

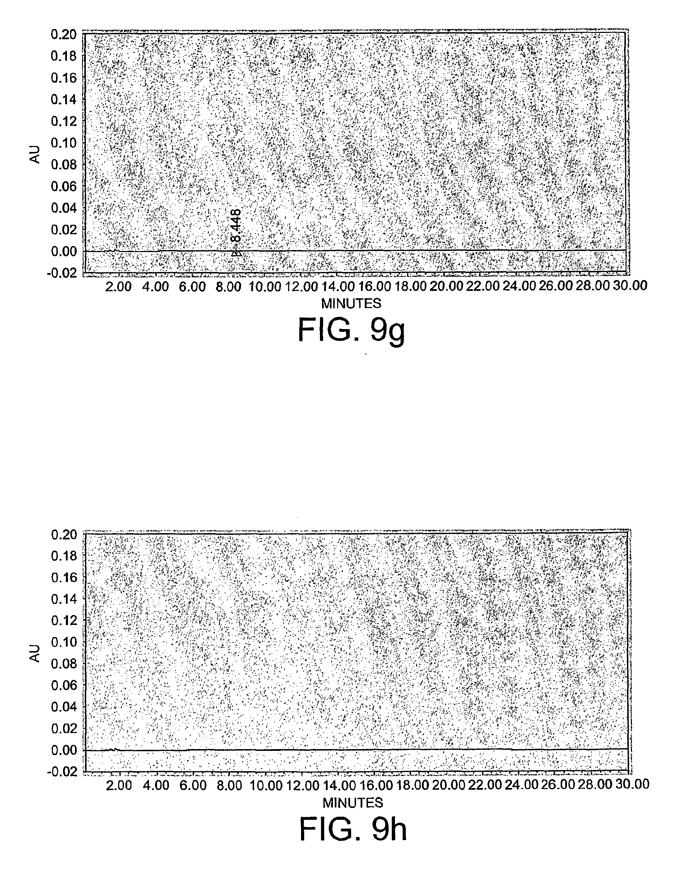

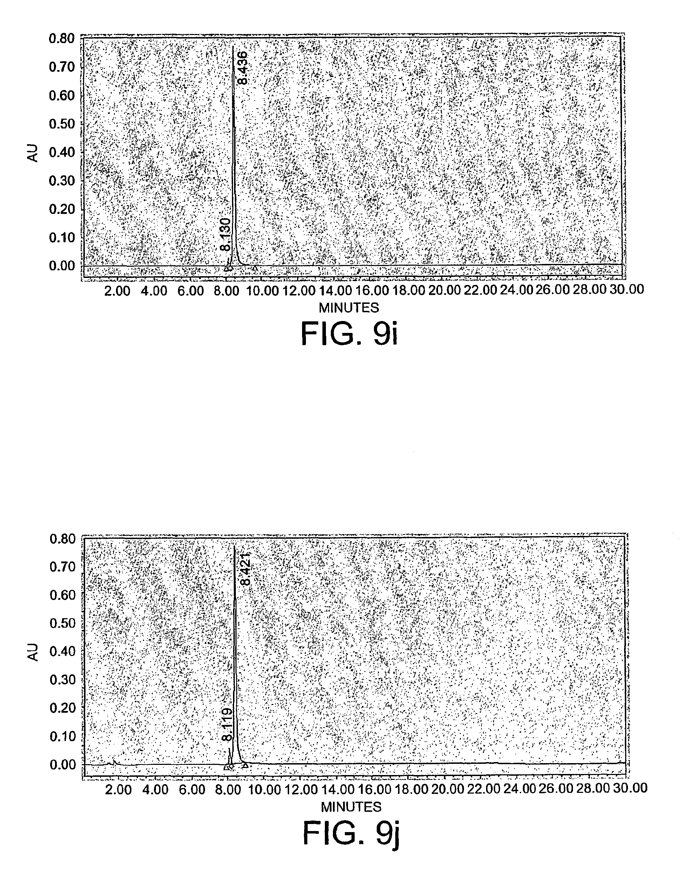

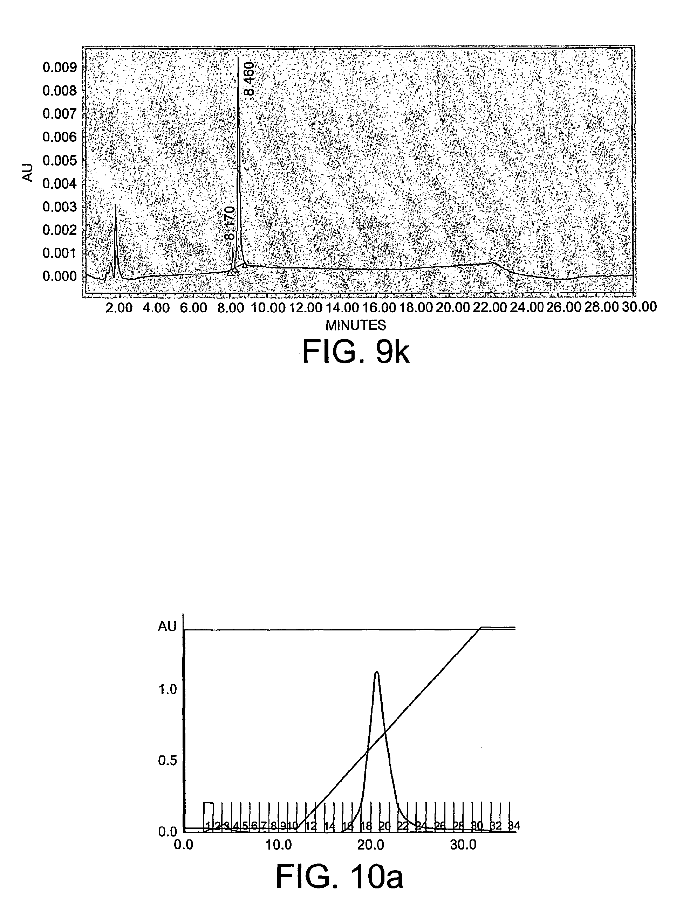

From the chromatograms several conclusions were drawn. After 20 minutes recirculation virtually no plasmid passed through the membrane (Fig 9a). The total amount of plasmid in the reservoir was 12.78 mg as compared to 16.33 mg in the start material (Fig 9b and 9c). It can therefore be inferred that 3.55mg plasmid is participating in the gel layer.

Visual inspection of the permeate samples (Fig 9d to 9h) showed contaminants passing through the membrane, but not plasmid. The chromatograms from the retentate harvest and the washes (Fig 9i to 9k) showed good clearance of contaminants.

82% of the total plasmid was recovered in the retentate harvest, and 13.7 % was recovered in the wash. This was significantly higher than the recoveries seen with an open channel cassette (typically 70-80 %) for the combined retentate harvest and wash.

When the process was applied to an RNase free process as previously described in the general protocol improved plasmid yields were also obtained.

Clearly the use of screened channels to improve plasmid yields has general application to any ultra filtration process and is an independent aspect of this application.

### EXAMPLE 9 (Effect of additional chromatography steps.)

Because the described RNase free process is particularly effective at removing low molecular weight RNA the applicant wished to determine whether other RNA removal processes, particularly those known to remove high molecular weight RNA might be used in combination with their RNase free process either as a pre or post ultrafiltration step (See fig 1b). They thus undertook the following experiment to determine the effect of a post ultrafiltration Calcium Chloride precipitation step.

Load material was prepared following the general protocol previously described (100mM Tris pH 7.5 buffer was used) to remove low molecular weight RNA during ultrafiltration. This was followed by a Calcium Chloride precipitation step. (1.4 M pH 7.5)

To one volume of retentate pool was added one volume of 2.8 M Calcium Chloride in 100 mM Tris pH 7.5. The mixture was stirred and incubated at room temperature for 10 minutes. The precipitated material was removed by filtration through a 0.22µm filter.

Calcium chloride salts are known to interfere with both tangential flow filtration and ion exchange chromatography. Therefore if a combined process were to prove satisfactory it would be necessary to effect removal of the calcium chloride. The applicant determined that reverse phase chromatography could be used to not only separate plasmid DNA from calcium chloride salts, but by selecting an appropriate ion pairing agent both the RNA and calcium chloride salts could be removed.

Investigation of various factors affecting the chromatography identified the ion pairing agent and the matrix as key to the separation of plasmid DNA from RNA.

If looking to remove only Calcium Chloride salts a simpler system could however be employed.

Two reversed phase media proved suitable in combination with tetra butyl ammonium chloride. They were:
Polyflow supplied by Puresyn, particle size 55 µm, which is non porous and is a proprietary polymer; and
Poros 50 R1 supplied by Poros, particle size 50µm, which is porous and is a polystyrene divinyl benzene polymer.

The preferred concentration of the tetra butyl ammonium chloride (TBAC) was 2 mM.

The preferred elution conditions for the two matrices varied:
For Polyflow, RNA removal was with 10 % ethanol and 2 mM TBAC and plasmid elution was with 30 % ethanol;
For Poros 50 R1, RNA removal was with 25 % ethanol and 2mM TBAC, and plasmid elution was with 40 % ethanol.

In both cases plasmid recovery was high (about 80%) and only very low levels of RNA could be detected.

Best results were obtained at low loads.

The application of reverse phase chromatography clearly shows itself to have potential in association with calcium chloride precipitation as both a RNA separation technique, irrespective of whether it is used in combination with the tangential flow ultra filtration method of the invention, and as a means of improving plasmid yields.

### EXAMPLE 10 (Use of hydroxyapatite chromatography)

A number of hydroxyapatite media were investigated to determine their ability to separate RNA from extra-chromosomal DNA. The effect of pH, buffer type, elution protocol, column capacity and flow rate were all investigated. The methodology was straight forward and comprised equilibrating a hydroxyapatite column with buffer at a given flow rate, loading a retentate pool (diluted in buffer) onto the column, washing with buffer, running a linear gradient and regenerating the column.

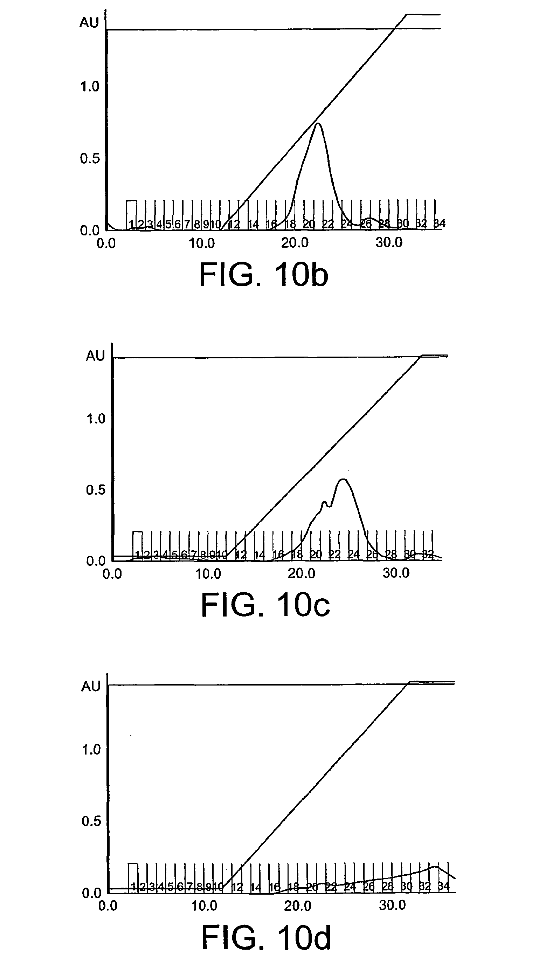

The significant findings from these experiments where as follows:
pH was found to be critical in successfully separating extra chromosomal DNA and RNA. For Macro Prep ceramic hydroxyapatite type II, 40 µm (Bio Rad) the optimum
pH was 7.8 (see fig 10c) and a difference of as little as 0.2 pH units had a significant effect on the separation. Thus at pH6.8 (fig 10a) and 8.8 (fig 10d) there was no separation whilst at 7.6 (fig 1 0b) resolution was significantly reduced.

Also, the buffer type proved significant with di sodium phosphate performing better than the more soluble di potassium phosphate buffer. In fact the best results were obtained using a double elution step combining the separation properties of di sodium phosphate with the high solubility of di potassium phosphate. The hydroxyapatite column was equilibrated and washed with 10 mM di sodium phosphate at pH 7.8, RNA was then eluted with 0.35 M di sodium phosphate at pH 7.8. Plasmid DNA was eluted with 0.2 M di potassium phosphate at pH 7.8 with a recovery of 78.6 % (table 1 below).

**Table 1**

| Buffer (elution 1) | Buffer (elution 2) | Peak | Volume | Plasmid Recovery (%) |
|---|---|---|---|---|
| 0.35M | 0.6M | 1 | 2.5 | 6.6 |
| Na₂HPO₄ | Na₂HPO₄ | 2 | 1.5 | 65.7 |
| pH 7.8 | pH 7.8 | | | |
| 0.35 M | 0.2M | 1 | 2.0 | 2.5 |
| Na₂HPO₄ | K₂HPO₄ | 2 | 1.0 | 78.6 |
| pH 7.8 | pH 7.8 | | | |
| 0.3M | 0.2M | 1 | 3.0 | 9.9 |
| Na₂HPO₄ | K₂HPO₄ | 2 | 1.0 | 76.2 |
| pH 7.8 | pH 7.8 | | | |

Replacing the RNA elution buffer with 0.3 M di sodium phosphate at pH 7.8 gave similar plasmid recoveries (76.2%) but RNA removal was not as effective. Replacing the plasmid elution buffer with 0.6 mM di sodium phosphate buffer at pH 7.8 resulted in lower plasmid recovery (65.7 %).

Loading the column with more than 0.5 mg total nucleic acid per ml of column using the step elution conditions noted in table 1 resulted in plasmid losses in the RNA elution peak of 56-58%.

At 0.5 mg/ml total nucleic acid loading capacity, a flow rate of 2ml/min also led to heavy plasmid loss of 7.7 % in the RNA elution fraction.

The optimal conditions were as follows:
1. Equilibrate and wash column in 10 mM Na₂HPO₄ pH 7.8
2. Elute RNA with 0.35 M Na₂HPO₄ pH 7.8
3. Elute plasmid DNA in 0.2 M K₂HPO₄ pH 7.8
4. Flow rate 0.5 ml/min.
5. Column capacity 0.5 mg total nucleic acid per ml of column or 25.4 mg plasmid per ml of column

These conditions gave rise to a plamid recovery of 78.6% and the product appeared free of RNA (using ion exchange chromatography).

Similar results were obtained using hydroxyapatite from Merck with the exception that the pH optimum was 7.5 and the Na₂HPO₄ buffer is best at 0.25M.The objective is to maximize binding of plasmid to increase column capacity. Since RNA binds to the column and is present in large amounts it takes a lot of the binding capacity of the hydroxapatite. However since the RNA has a lower affinity for the column than plasmid, conditions of high phosphate are used to prevent RNA binding to the column and as a result maximise plasmid binding. These conditions are 0.35 M Na₂HPO₄ pH 7.8 for BioRad and 0.25 M Na₂HPO₄ pH 7.5 for Merck. Under these conditions capacities of 300-500µg /ml can be achieved.
The alternative routes illustrated with reference to figure 1b give rise to plasmid DNA of differing degrees of purity and different yields.
Thus, for example, the process illustrated as route B gave rise to the removal of up to 96% of endotoxins and up to 99.89% genomic DNA whereas the process illustrated as route C gave rise to the removal of up to 99.97 % genomic DNA.

The selection of any particular process will therefore depend on a number of factors.
Whilst in the previous examples the ultrafiltration step was conducted in accordance with the RNase-free plasmid purification protocol (300g scale outlined earlier) and involved forming a gel layer by recirculation for 30 minutes, the applicant has determined that recirculation is not necessary. This is demonstrated in Example 11 below.

### EXAMPLE 11 (UF, without recirculation)

### a) Control (with RNase)

RNase was added during alkaline lysis and the clarified lysate loaded onto an ultrafiltration membrane without recirculation.

| | | |
|---|---|---|
| Construct | = | 7.8 kb plasmid |
| Membrane | = | 300K |
| Plasmid load | = | 183 mg/ft² |
| TMP | = | 10 psi |
| Diafiltration buffer | = | 50 vol 10 mMTris pH8 |
| | | |
| Results | Plasmid recovery = 52% | |
| | Plasmid loss during concentration / diafiltration = 38% | |

### b) Comparison (RNase free process)

The construct, membrane & diafiltration buffer were as stated for the control. The plasmid loading varied (as shown) & results were obtained at a TMP of both 5 & 10 psi. See table 2 below.

**Table 2**

| | Plasmid load | Plasmid recovery | Plasmid losses | SEC-HPLC |
|---|---|---|---|---|
| TMP = 5 psi | 116mg/ft2 | 52% | 3% | NA |
| TMP=10 psi | 144mg/ft2 | 90% | 0 | 83% RNA |

The significant difference in plasmid loses between a) control, and b) the comparison for a RNase free process indicate recirculation is not necessary in a RNase free process.

Without wishing to be bound by theory, it is possible that the presence of high levels of RNA in the RNase free process result in the formation of an 'instant' gel layer possibly due to the high levels of RNA. In this regard, it should be noted that the lysate in the RNase free process contains ~98% by weight RNA to ~2% by weight extra chromosomal DNA.

Indeed, the hypothesis is supported by the results obtained in Example 12 below, in which the effect of plasmid load concentration was studied.

### EXAMPLE 12 (Effect of load concentration)

In this example, the effect of reducing plasmid load was studied. The results are indicated in table 3 below.

**Table 3**

| | Plasmid load | Plasmid recovery | Plasmid losses | SEC-HPLC |
|---|---|---|---|---|
| 2 x dilution | 86mg/ft2 | 72% | 0.7% | 78% RNA |
| 4 x dilution | 42mg/ft2 | 86% | 1.3% | 65% RNA |
| 8 x dilution | 19mg/ft2 | 82% | 7% | 46% RNA |
| 16 x dilution | 6mg/ft2 | 141% | 5% | 34% RNA |

What is evident from the results is that by diluting both plasmid & RNA in the lysate load, plasmid losses increase although plasmid recovery remain high.

In view of the results obtained in Examples 11 and 12, the applicant decided to investigate whether recirculation was necessary in a 2^{nd} stage ultrafiltration (UF₂) i.e. one in which most of the RNA had previously been removed. The protocol and results are given in Example 13 below.

### EXAMPLE 13 (Recirculation for 2^{nd} Step UF)

In this experiment the effect of:
i) The concentration of plasmid in the start material
ii) The TMP and
iii) The plasmid size
were studied to determine how they affected plasmid recovery

The UF₂ was employed to reduce the volume of a post TMAE pool i.e. one which had undergone a purification step using chromotography.

The materials were as listed below:

### Materials

Post TMAE pool containing plasmid (7.7Kbp) in 50mM Tris/0.74M NaCl pH 8.5, stored at 2-8°C.

Post TMAE pool containing plasmid (5.5Kbp) in 50mM Tris/0.74M NaCl pH 8.5, stored at 2-8°C.

The buffer used as a diluant was 50mM Tris/0.74M NaCl pH 8.5

The ultrafiltration cassette was cleaned using 0.5M NaOH and stored in 0.1M NaOH according to the manufacturer's instructions.

### Experimental

TMAE eluate was diluted with 50mM Tris/0.74M NaCl pH 8.5 to the required concentration. Samples were taken for HPLC analysis (Dionex) and A260 measurement. 500ml was placed in the reservoir with the remainder being placed in a bottle connected to the 500ml reservoir. The pump was switched on to give a cross flow rate (QR) of 900ml/min. The retentate valve was closed slightly to give the required trans-membrane pressure (TMP). The permeate line was placed in a clean bottle, and the lysate was concentrated to a final volume of 250ml. In total, four fractions of permeate were collected (volumes dependent on the plasmid concentration of the start material). Samples were taken for HPLC analysis (Dionex) and A260 measurement.

The retentate valve was opened and the permeate line clamped to remove TMP. The system was left to recirculate for 10 minutes to dissolve any plasmid associated with the membrane and harvest the plasmid. After 10 minutes, the UF2 retentate was harvested from the reservoir and replaced with 100 ml 50mM Tris/0.74M NaCl pH 8.5. This was recirculated for 10 minutes as before, harvested into a separate container and labelled 'Wash 1'. The wash step was repeated twice and the harvests labelled 'Wash 2' and 'Wash 3'. The volumes of UF2 retentate, Wash 1, Wash 2 and Wash 3 were measured and samples taken for HPLC analysis (Dionex) and A260 measurement.

The experiments were conducted under the conditions noted in Table 4 below.

**Table 4**

| Experiment No | Plasmid Conc. (µg/ml) | TMP (psi) | Plasmid size |
|---|---|---|---|
| 064 | 200 | 10 | 7.7 kbp |
| 065 | 400 | 10 | 7.7 kbp |
| 066 | 600 | 10 | 7.7 kbp |
| 068 | 100 | 10 | 7.7 kbp |
| 069 | 400 | 15 | 7.7 kbp |
| 070 | 300 | 10 | 5.5 kbp |
| 071 | 250 | 15 | 5.5 kbp |
| 072 | 200 | 20 | 5.5 kbp |

The results are given in Table 5 below:

| Experiment No | Recovered (%) | Lost (%) | Unaccounted (%) |
|---|---|---|---|
| 064 | 86.25 | 2.95 | 10.79 |
| 065 | 90.94 | 3.70 | 5.36 |
| 066 | 93.05 | 3.08 | 3.87 |
| 068 | 91.73 | 5.58 | 2.70 |
| 069 | 85.83 | 5.33 | 8.84 |
| 070 | 95.54 | 3.53 | 0.93 |
| 071 | 86.30 | 5.85 | 7.85 |
| 072 | 86.10 | 6.50 | 7.40 |

The results suggest polarisation of the ultrafiltration membrane is unnecessary.

Since, in an RNase free extra chromosomal DNA purification process the levels of RNA remaining after alkaline lysis are particularly high (about 20 times the amount of plasmid DNA by weight) the applicant looked at ways of reducing RNA levels prior to ultrafiltration.

Referring to Fig 1b 'c', it will be apparent that the applicant had already proposed using CaCl₂ prior to the ultrafiltration step. This step removed significant amounts of RNA and consequently further investigations were made with a view to optimising the ultrafiltration step in the presence of calcium chloride. These investigations are set out in Example 14.

### EXAMPLE 14 (CaCl₂ & pore size)

Since previous experiments with CaCl₂ suggest significant amounts of plasmid were lost through a 300K membrane the applicant hyphthesised that the CaCl₂ might be 'condensing' the plasmid. They therefore tried using a membrane with a smaller pore size (100K). However this alone gave a plasmid recovery lower than expected. This was rectified using a lower TMP (5 psi vs 10 psi).
Separation of the cell debris was made:
a) using centrifugation and
b) using a sieve

Comparative experiments with and without recirculation are shown below:
**a) UF1 loading lysate post calcium chloride precipitation with recirculation**
   Clarified lysate was produced by alkaline lysis in a 50g preparation.
   1 volume of 2M CaCl₂ stock solution was added to 1 volume of clarified lysate under stirring. The mixture was incubated at room temperature for 10 minutes. The precipitate was centrifuged out at 5000rpm for 10 minutes. The supernatant was passed through a 0.45/0.2µm Sartobran 300 filter.
   An ultrafiltration system consisting of a Pall Filtron Centramate rig fitted with a 1ft² 100K Omega Centramate Open Channel membrane was emptied of storage solution and washed with 10mM Tris pH8.5.
   The reservoir was filled with approximately 400ml of clarified lysate from a 50g prep and connected to a bottle containing the rest of the lysate.
   Recirculation was carried out by connecting the permeate line to the reservoir. The Watson Marlow pump was set at 158rpm (cross flow rate of 1L/min/ft²), and the retentate valve was adjusted to obtain a TMP of 5psi. The recirculation lasted 20 minutes during which time the TMP was not allowed to rise above 10psi.
   The clarified lysate was concentrated to 75ml by connecting the permeate line to a 2L bottle (concentration permeate).
   Diafiltration was started by connecting the reservoir to a bottle of 10mM Tris pH8.5 solution so that filtrate lost was replaced by fresh buffer at the same rate. Diafiltration permeate was collected in 5x1L bottles. 30 volumes (3L) of 10mM Tris pH8.5 was followed by 20 volumes (2L) of 50mM Tris + 0.54M NaCl pH8.5.
   At the end of diafiltration, the retentate valve was opened (no back pressure), the permeate line was closed and the contents of the UF system were recirculated for 30 minutes. The UF1 diafiltered concentrate was recovered by opening the retentate line (retentate harvest).
   The reservoir was filled with 100ml of 50mM Tris + 0.54M NaCl pH8.5 and recirculated for 30 minutes. The UF1 diafiltered concentrate was recovered by opening the retentate line (retentate wash 1). This was repeated one more time (retentate wash 2).
   UF1 retentate harvest and 2 washes were pooled and passed through a 0.45/0.2µm Sartobran 300 filter.
   The UF1 system was then sanitised by recirculating 300ml of 0.5M NaOH for 30 minutes.
   The UF1 system was stored in 0.1M NaOH.
   The experiment was repeated but clarified lysate from 2x50g preps was pooled, calcium chloride precipitated and processed through UF1 so that the UF1 load was doubled.
**b) UF1 loading lysate post calcium chloride precipitation without recirculation**
   i) Using centrifugation to remove cell debris
      Clarified lysate was produced by alkaline lysis in a 100g preparation
      1 volume of 2M CaCl₂ stock solution was added to 1 volume of clarified lysate under stirring. The mixture was incubated at room temperature for 10 minutes. The precipitate was centrifuged out at 4600rpm for 20 minutes. The supernatant was passed through a 5µm Sartopure PP2 followed by a 0.45/0.2µm Sartobran P filter.
      An ultrafiltration system consisting of a Pall Filtron Centramate rig fitted with a 1ft² 100K Omega Centramate Open Channel membrane was emptied of storage solution and washed with 10mM Tris pH8.5.
      The reservoir was filled with approximately 400ml of clarified lysate from a 100g prep and connected to a bottle containing the rest of the lysate. The Watson Marlow pump was set at 158rpm (cross flow rate of 1L/min/ft²) and the retentate valve was adjusted to obtain a TMP of 5psi. The clarified lysate was concentrated to 75ml and the concentration permeate collected in a 2L bottle. The TMP was not allowed to rise above 10psi.
      Diafiltration was started by connecting the reservoir to a bottle of 10mM Tris pH8.0 buffer so that filtrate lost was replaced by fresh buffer at the same rate. Diafiltration permeate was collected in 5x1L bottles. 30 volumes (3L) of 10mM Tris pH8.0 was followed with 20 volumes (2L) of 50mM Tris + 0.54M NaCl pH8.5.
      At the end of diafiltration, the retentate valve was opened (no back pressure), the permeate line was closed and the contents of the UF system were recirculated for 30 minutes. The UF1 diafiltered concentrate was recovered by opening the retentate line (retentate harvest).
      The reservoir was filled with 75ml of 50Mm Tris + 0.54M NaCl pH8.5 and recirculated for 30 minutes. The UF1 diafiltered concentrate was recovered by opening the retentate line (retentate wash 1). This was repeated one more time (retentate wash 2).
      UF1 retentate harvest and 2 washes were pooled and passed through a 0.45/0.2µm Sartobran 300.
      The UF1 system was then sanitised by recirculating 300ml of 0.5M NaOH for 30 minutes.
      The UF1 system was stored in 0.1 M NaOH.
   ii) Using filtration to remove cell debris
      Plasmid construct:5.9kbp *E. coli* host cell line:.
      100g of bacterial cell paste stored at -70°C was broken down to small pieces with a mallet. 500ml of resuspension buffer was added to a Bellco resuspension vessel at room temperature. The broken cell paste was added to the vessel and stirred for 1 hour at room temperature at a magnetic stirrer setting of 8.
      Immediately prior to lysis, 834ml of cold 0.96% NaOH was added to 166ml of 6% SDS in the cold room. The cell suspension was transferred to a 4L beaker with a Heidolph overhead stirrer and into the cold room. The lysis mixture was added to the cell resuspension and stirred for 30min at 40rpm.
      500ml of 3M potassium acetate was added to the lysate and stirred for 30min at 60rpm at 4°C.
      A 20ml sample of lysate was taken and centrifuged at 2500rpm for 10min on a MSE Mistral 2000. The supernatant was the clarified lysate.
      To 1 volume of lysate, 0.66 volume of 3M CaCl₂ stock solution was added under stirring. The mixture was left to stand for 10 minutes at room temperature.
      The mixture was stirred again and poured onto a Plastok holder fitted with a 350mm diameter, 200µm pore size mesh filter. The filtrate was recovered in 5L beaker under gravity.
      The filtrate post mesh was pumped through a Millipore CE 15 depth filter cartridge and filtrate was collected in a 5L beaker.
      The filtrate post depth filter was pumped through a 0.45/0.2µm Sartobran P filter. This material was called the lysate post calcium chloride precipitation.
      The lysate post calcium chloride precipitation was processed through UF1 as previously except that the diafiltration was against 35 volumes of 10mM Tris pH8.0 followed by 15 volumes of 10mM Tris + 0.45M NaCl pH8.5.
      The experiment was repeated but the lysate was diluted 2x with UF1 concentration permeate before the addition of calcium chloride solution.

### Desalting

All samples containing calcium chloride salt (including lysate post calcium chloride precipitation, UF1 concentration permeate and UF1 diafiltration permeate) were desalted before analysis by HPLC.

2ml of sample was injected on 2x5ml HiTrap Desalting columns in series equilibrated with 50mM Tris pH8.5 at a flow rate of 5ml/min and a 4.5ml desalted peak of absorbance at 254nm was collected.

### Analysis by HPLC

Plasmid concentration was determined by ion exchange-HPLC on a Dionex DNAPac column.

RNA peak area was determined by size exclusion-HPLC on a TosoHaas TSK gel G-DNA-PW in series with a G3000SWXL column. Running buffer: 0.1M Tris + 0.3M NaCl + 1mM EDTA pH7.5. Flow rate = 0.3ml/min. RNA content is the amount of RNA as a percentage of nucleic acids.

### Results

### a) UF1 loading lysate post calcium chloride precipitation with recirculation

The results are shown in table 6 below which shows: effect of increased nucleic acid load on the performance of UF1 post calcium chloride precipitation.

**Table 6.**

| **UF1 load (mg plasmid/ft²)** | **Plasmid recovery (IE-HPLC)** | **RNA content (SEC-HPLC)** |
|---|---|---|
| 60.0 | 109.9% | 2.61% |
| 108.0 | 121.5% | 4.51% |

Previous experiments on a Millipore Labscale TFF system fitted with a low volume Centramate 0.1ft² 300K Omega membrane suggested that, in the presence of calcium chloride salt, significant amounts of plasmid were lost through the membrane. A 100K membrane was therefore used instead with no significant plasmid losses. However, plasmid recovery was lower than expected but this was rectified by using a lower starting TMP of 5psi instead of 10psi. RNA clearance was maximised, as in the UF1 without calcium chloride, by using a diafiltration buffer with a low ionic strength.

A 1ft² Centramate system was used to confirm the Labscale results, loading 60.0mg of plasmid per square foot of membrane in the presence of 1M calcium chloride salt. Plasmid recovery was high at over 100% and RNA clearance was very effective with only 2.61% RNA content in the UF1 retentate (table 6). Contrary to the UF1 without calcium chloride, RNA was cleared during concentration and the first 10 diafiltration volumes.

The membrane load was then increased to 108mg plasmid/ft² with similar results in plasmid recovery (>100%) and RNA clearance (4.51% content in the UF1 retentate).

Diafiltration was conducted in two steps: first, 30 volumes of 10mM Tris pH8.5 was used to maximise RNA clearance. This was followed by 20 volumes of 50mM Tris + 0.54M NaCl pH8.5 to buffer exchange the plasmid and prepare it for direct loading on to the Fractogel TMAE column.

### b) UF1 loading lysate post calcium chloride precipitation without recirculation

UF1, without recirculation to build up a gel layer was conducted with lysate treated with calcium chloride. Only very small amounts of plasmid were detected in the permeate (0.2%) and plasmid recovery was very high (over 100%). The RNA content in the UF1 retentate was 4.64% (table 7). Table 7: illustrates the effect of the nucleic acid concentration in the load on the performance of UF1 without recirculation in the presence of calcium chloride.

**Table 7**

| **UF1 load (mg plasmid/ft²)** | **Plasmid conc (µg/mt)** | **Plasmid recovery** | | | **RNA content (SEC-HPLC)** |
|---|---|---|---|---|---|
| | | **Conc permeate** | **Diaf permeate** | **UF1 retentate** | |
| 126.5* | 69.9 | 0.2% | 0 | 108.8% | 4.64% |
| 109.3** | 60.7 | 1.3% | 0 | 90.5% | 0 |
| 129.6** | 36.0 | 5.9% | 7.2% | 82.7% | 1.93% |

| | | | | | |
|---|---|---|---|---|---|
| plasmid construct: *7.7kbp, **5.9kbp | | | | | |

The cell debris removal step post neutralisation and the calcium chloride precipitate removal step were combined. A calcium chloride stock solution of 3M was used to minimise process volumes. 0.66 volume of the stock solution was added directly to 1 volume of lysate post neutralisation. The mixture was passed through a mesh filter to remove cell debris then through a depth filter to remove finer particles. This process was quicker than centrifugation and is also more easily contained. The second diafiltration buffer was also changed from containing 0.54M NaCl to 0.45M NaCl. The plasmid construct was changed to a smaller size from (7.7Kbp) to (5.9Kbp).

Plasmid losses in the concentration permeate were higher at 1.3%which may be due to the smaller plasmid size. Plasmid recovery was 90.5% and no RNA was detected in the UF1 retentate (table 7).

The lysate was then diluted 2 times with concentration permeate from a previous run before the addition of calcium chloride so that the plasmid concentration in the UF1 load was reduced from 60.7 to 36µg/ml. Plasmid losses in the concentration permeate significantly increased to 5.9% and the plasmid recovery was lower at 82.7%. This could indicate that smaller plasmids from a low producing host strain may give lower recoveries at the UF1 stage.

A number of conclusions can be drawn from the results of the experiments outlined in Example 14.
1. RNA appeared to contribute to the gel layer during recirculation so that only low levels were cleared through the membrane during concentration.
2. The ionic strength of the diafiltration buffer was the most important factor in the removal of RNA: a low conductivity significantly increased the passage of RNA through the membrane during diafiltration although the exact mechanism is not understood.
3. 50 diafiltration volumes is recommended to give thorough clearance of RNA.
4. A membrane pore size of 300K is recommended for plasmid sizes of 5Kbp or above.
5. The other important factor in the removal of RNA was the nucleic acid load: a lower load resulted in higher clearance.
6. The addition of calcium chloride to the lysate to precipitate RNA required the modification of the conditions used for the UF1 step.
   i) A smaller, e.g.100K membrane was necessary to prevent plasmid losses.
   ii) A lower TMP (5psi) was also desirable to improve plasmid recovery.

After precipitation with calcium chloride, only low molecular weight RNA was left and this was removed efficiently by the ultrafiltration step.

7. Recirculation to polarise the membrane was found not to be necessary in a RNase-free process in the presence or absence of calcium chloride. This may be due to the presence of large amounts of RNA which quickly polarise at the beginning of concentration. However, reducing the nucleic acid load did not produce the losses of plasmid observed when RNase was added during lysis and recirculation was not carried out. In the presence of calcium chloride, a smaller plasmid construct was processed through the UF1 at half the normal concentration with only minimal losses of plasmid through the membrane. This shows the robustness of the UF1 step in the presence of calcium chloride without recirculation.

In view of the above findings the applicant went on to see if they could further optimise a process using, as an example of an antichaotropic salt, CaCl₂. They studied 3 alternative regimes and the results of these investigations are given in Example 15 below:

### EXAMPLE 15

The three regimes studied where:

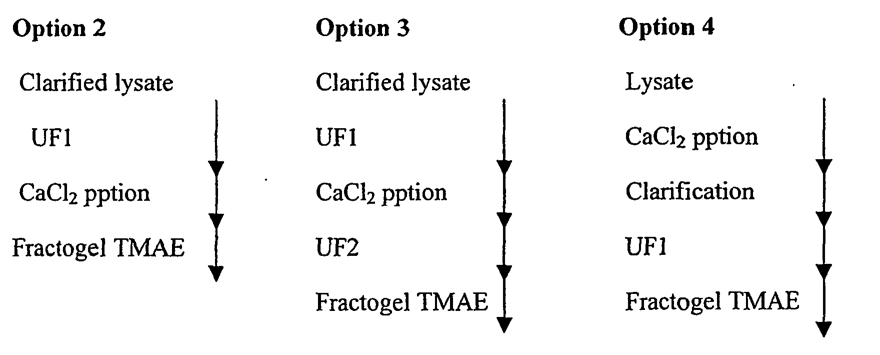

### Methods

### Lysis

100g of bacterial cell paste stored at -70°C was broken down to small pieces with a mallet. 500ml of resuspension buffer was added to a Bellco resuspension vessel at room temperature. The broken cell paste was added to the vessel and stirred for 1 hour at room temperature at a magnetic stirrer setting of 8.
Immediately prior to lysis, 834ml of cold 0.96% NaOH was added to 166ml of 6% SDS in the cold room. The cell suspension was transferred to a 3L beaker with a Heidolph overhead stirrer and into the cold room. The lysis mixture was added to the cell resuspension and stirred for 30min at 40rpm.
500ml of 3M potassium acetate was added to the lysate and stirred for 30min at 80rpm at 4°C.

The lysate was centrifuged at 8000g for 10min on a Sorvall RC5C Plus centrifuge. The supernatant was filtered through a double layer of Miracloth then passed through a 5µm Sartopure PP2 filter followed by a 0.45/0.2µm Sartobran P. This material was called the clarified lysate.

### UF1

An ultrafiltration system consisting of a Pall Filtron Centramate rig fitted with a 1ft² 300K Omega Centramate Open Channel membrane was emptied of storage solution and washed with diafiltration buffer (10mM Tris pH8.0).

The reservoir was filled with approximately 500ml of clarified lysate and connected to a bottle containing the rest of the lysate. Recirculation was carried out by connecting the permeate line to the reservoir. The WatsonMarlow pump was set at 155rpm (cross flow rate of 1L/min/ft²), and the TMP set at 5psi. The recirculation lasted 20 minutes during which time the TMP was not allowed to rise above 10psi.

The clarified lysate was concentrated to 75ml by connecting the permeate line to a 2L bottle (concentration permeate).

Diafiltration was started by connecting the reservoir to the diafiltration buffer bottle so that filtrate lost was replaced by fresh buffer at the same rate. Diafiltration permeate was collected in 5x1L bottles. 50 volumes (5L) of diafiltration buffer was used.

At the end of diafiltration, the retentate valve was opened (no back pressure), the permeate line was closed and the contents of the UF system were recirculated for 30 minutes. The UF1 diafiltered concentrate was recovered by opening the retentate line (retentate harvest).

The reservoir was filled with 75ml of fresh diafiltration buffer and recirculated for 30 minutes. The UF1 diafiltered concentrate was recovered by opening the retentate line (retentate wash 1). This was repeated one more time (retentate wash 2).

UF1 retentate harvest and 2 washes were pooled and passed through a Gelman Science 0.45µm filter.

The UF1 system was then sanitised by recirculating 500ml of 0.5M NaOH for 1 hour.
The UF1 system was stored in 0.1M NaOH.

### Calcium chloride precipitation

To 1 volume of UF1 retentate, 0.12 volume of 2.8M CaCl₂ in 0.1M Tris pH8.0 was added so that the final calcium chloride concentration was 0.3M. The mixture was incubated for 10 minutes at room temperature.

The mixture was then diluted 2x with 0.1M Tris pH8.5 to make the final calcium chloride concentration O.15M. The precipitate was then filtered out by passing through 3x 25CE filters followed by 4x 40CE filters and finally 2x 0.45/0.2µm Sartobran P filters.

This material was called the UF1 post calcium chloride precipitation.

### Fractogel TMAE anion exchange chromatography

A 20ml Fractogel TMAE column (1.6x10cm) was equilibrated with 10 column volumes of 50mM Tris pH8.5 at a flow rate of 5.1 ml/min (152cm/h).

The column was loaded at about 2.5mg plasmid per ml of column with diluted UF1 post calcium chloride precipitation. The column was washed with 2 column volumes of 50mM Tris pH8.5 followed by 5 column volumes of 50mM Tris + 0.54M NaCl pH8.5.

Plasmid was eluted with a 10 column volume gradient of 0.54 to 3M NaCl in 50mM Tris pH8.5.

The column was regenerated with 4 column volumes of 50mM Tris + 3M NaCl pH8.5.

The column was re-equilibrated with 5 column volumes of 50mM Tris pH8.5.

### UF2

An ultrafiltration system consisting of a Millipore Labscale system fitted with a 0.1ft² 100K Omega Centramate Open Channel membrane was emptied of storage solution and washed with diafiltration buffer (10mM Tris pH8.0).

The reservoir was filled with undiluted UF1 post calcium chloride material. Recirculation was carried out by connecting the permeate line to the reservoir. The pump was set at 4 (cross flow rate of 1 L/min/ft²), and the TMP set at 5psi. The recirculation lasted 20 minutes during which time the TMP was not allowed to rise above 10psi.

The UF1 post calcium chloride was concentrated to 35ml by connecting the permeate line to a 500ml bottle (concentration permeate).

Diafiltration was started by connecting the reservoir to a bottle of 10mM Tris pH8.0 buffer so that filtrate lost was replaced by fresh buffer at the same rate. Diafiltration was against 40 volumes of 10mM Tris pH8.0 followed by 10 volumes of 50mM Tris + 0.54M NaCl pH8.5. Diafiltration permeate was collected in 4x500mL bottles.

At the end of diafiltration, the retentate valve was opened (no back pressure), the permeate line was closed and the contents of the UF system were recirculated for 30 minutes. The UF2 diafiltered concentrate was recovered by opening the retentate line (retentate harvest).

The reservoir was filled with 50ml of 50mM Tris + 0.54M NaCl pH8.5 and recirculated for 30 minutes. The UF1 diafiltered concentrate was recovered by opening the retentate line (retentate wash 1). This was repeated one more time (retentate wash 2).

UF1 retentate harvest and 2 washes were pooled and passed through a Sterivex GV filter.

The UF1 system was then sanitised by recirculating 100ml of 0.5M NaOH for 1 hour.
The UF1 system was stored in 0.1M NaOH.

### Process option 2

Alkaline lysis was conducted as above, followed by UF1 as above, calcium chloride precipitation as above. 237ml of UF1 post calcium chloride precipitation was loaded on the Fractogel TMAE column. 240ml breakthrough and wash was collected (peak 1) as well as 75ml eluate (peak 2).

280ml UF1 retentate was produced but only 210ml was recovered post filtration. Some material was lost on the filters and some was lost due to leakage.

482ml of diluted UF1 retentate post calcium chloride precipitation was produced. Only 400ml was recovered post filtration.

### Process option 3

This option is similar to 2 in that alkaline lysis was conducted followed by UF1 as and calcium chloride precipitation except that the UF1 retentate post calcium chloride precipitation was not diluted.

An ultrafiltration step (UF2) was introduced to remove calcium chloride salts which otherwise interfere with the chromatography column.

90ml of UF2 retentate was loaded on a 20ml Fractogel TMAE column as above except that the 10 column volume gradient was 0.54-1M NaCl in 50mM Tris pH8.5.

315ml of UF1 retentate was produced but only 295ml was recovered post filtration.

330ml of UF1 post calcium chloride precipitation was produced but only 320ml was recovered post filtration.

### Process option 4

### Lysis

The lysis step was as previously described except that it was not centrifuged as previously described. Instead the lysate was subjected to calcium chloride precipitation and cell debris removal as set out below:

### Calcium chloride precipitation and cell debris removal

A 20ml sample of lysate was centrifuged on a MSE Mistral 2000 at 2500rpm for 10 minutes. The supernatant was called the clarified lysate.

To 2100ml of lysate, 1960ml of 2M CaCl₂ solution was added under stirring and then allowed to stand at room temperature for 10 minutes.

The mixture was poured on to a Plastok holder fitted with a 350mm, 200µm mesh filter to remove cell debris and the filtrate was collected into a 5L beaker under gravity. The filtrate was then passed through a Millistak 15CE cartridge depth filter followed by a 0.45/0.2µm Sartobran P filter. The filtrate was called the lysate post calcium chloride (vol = 3350ml).

### UF1

An ultrafiltration system consisting of a Pall Filtron Centramate rig fitted with a 1ft² 100K Omega Centramate Open Channel membrane was emptied of storage solution and washed with 10mM Tris pH8.0 diafiltration buffer.

The reservoir was filled with approximately 500ml of lysate post calcium chloride and connected to a bottle containing the rest of the lysate. Recirculation was carried out by connecting the permeate line to the reservoir. The Watson Marlow pump was set at 155rpm (cross flow rate of 1L/min/ft²), and the TMP set at 5psi. The recirculation lasted 20 minutes during which time the TMP was not allowed to rise above 10psi.

The clarified lysate was concentrated to 75ml by connecting the permeate line to a 2L bottle (concentration permeate).

Diafiltration was started by connecting the reservoir to a bottle of 10mM Tris pH8.0 buffer so that filtrate lost was replaced by fresh buffer at the same rate. Diafiltration was carried out with 35 volumes of 10mM Tris pH8.0 followed by 15 volumes of 50mM Tris + 0.54M NaCl pH8.5. 5x1L samples of diafiltration permeate were collected.

At the end of diafiltration, the retentate valve was opened (no back pressure), the permeate line was closed and the contents of the UF system were recirculated for 30 minutes. The UF1 diafiltered concentrate was recovered by opening the retentate line (retentate harvest).

The reservoir was filled with 100ml of 50mM Tris + 0.54M NaCl pH8.5 and recirculated for 30 minutes. The UF1 diafiltered concentrate was recovered by opening the retentate line (retentate wash 1). This was repeated one more time (retentate wash 2).

UF1 retentate harvest and 2 washes (vol = 290ml) were pooled and passed through a Gelman Science 0.45□m filter (final vol = 270ml).

### Fractogel TMAE anion exchange chromatography

This was as previously described except the 10 column volume gradient was 0.54-1M NaCl in 50mM Tris pH8.5.
125ml of UF1 retentate was loaded on the column. 135ml of breakthrough and wash was collected (peak 1) as well as 135ml of eluate (peak 2).

### The samples were subjected to analysis as set out below:

### Sample analysis:

### Ion exchange-HPLC

Plasmid concentration was determined by anion exchange-HPLC on a Dionex DNAPac PA-100 4x250mm column following the standard protocol. Samples were digested with RNase to remove any interference of RNA: 2µl of bovine pancreas RNase was added to 100µl of sample.

Plasmid topology was determined by anion exchange-HPLC on a TSK DNA-NPR 4.6mmx7.5cm, 2.5µm column following the standard protocol.

### RNA assay

RNA concentration was determined by BAD using a reverse phase-HPLC based assay.

Plasmid/RNA ratio was determined by size exclusion-HPLC on a TSK G-DNA-PW 7.8mmx30cm, 10µm column coupled to a TSK G3000 SWXL 7.8mmx30cm, 5µm column. The mobile phase was 0.1M Tris + 0.3M NaCl + 1mM EDTA pH7.5. Flow rate was 0.3m1/min. Sample injection was 100µl.

### Protein assay

Protein concentration was determined using the Micro BCA protein assay reagent kit from Pierce. A standard curve was plotted using BSA solution in the range 0.625-20µg/ml. Samples were suitably diluted with PBS to fall within the range of the BSA standards.

### Endotoxin assay

Endotoxin concentration was determined by Pharmaceutical Microbiology using a standard LAL assay.

### Genomic DNA assay

Genomic DNA concentration was determined by BAD using a quantitative PCR assay.

### Desalting

Samples containing high levels of calcium chloride salt (lysate post calcium chloride precipitation, UF1 concentration permeate, UF1 diafiltration permeate) were desalted prior to analysis. Process 2 and 3 samples containing calcium chloride salt were not desalted since the low concentration was not interfering with the assays.

2ml of sample was injected on 2x5ml HiTrap Desalting columns in series equilibrated with 50mM Tris pH8.5 at a flow rate of 5ml/min and a 4.5ml desalted peak of absorbance at 254nm was collected.

### RESULTS AND DISCUSSION

The volumes used for all calculations were corrected for losses that occurred during filtration. This allowed for better comparison between each option.

### Process option selection: option 2

The clarified lysate was processed through a modified UF1 step to maximise removal of RNA. This included diafiltering with 50 volumes of a low ionic strength buffer and using a starting TMP of 5psi. The plasmid loading on the 1ft², 300K PES membrane was 136.4 mg of plasmid per square foot. The remaining RNA was then precipitated with 0.3M calcium chloride for 10 minutes at room temperature. The mixture was diluted 1 in 1 to a calcium chloride salt concentration of 0.15M and the precipitate was removed by filtration. The filtrate was then loaded directly on to a Fractogel TMAE anion exchange column. The dilution was necessary to prevent the calcium chloride salt from interfering with the binding of plasmid to the column. The column was loaded at 2.47 mg of plasmid per ml of column, washed with 50mM Tris to prevent early plasmid elution followed by 50mM Tris + 0.54M NaCl to elute the remaining RNA. Plasmid was eluted with a salt gradient.

Overall plasmid recovery was high at 89.2% (See table 8 below).

**Table 8**

| | **Vol (ml)** | **Plasmid conc (□g/ml)** | **Step recovery** | **Overall recovery** |
|---|---|---|---|---|
| **Lysate** | 1800 | 75.8 | NA | NA |
| UF1 conc permeate | 1700 | 0 | 0 | 0 |
| UF1 diafiltration permeate | 5000 | 0 | 0 | 0 |
| **UF1 retentate** | 280 | 381.8 | 78.3% | 78.3% |
| **UF1 post CaCl₂ pption** | 480 | 208.7 | 124.9% | 97.9% |
| TMAE peak 1 | 240 | 0 | 0 | 0 |
| **TMAE peak 2** | 75 | 601.1 | 91.1% | 89.2% |

The UF1 step gave a recovery of 78.3% while the recovery of the calcium chloride precipitation step was greater than 100%. The recovery across the chromatography step was 91.1%.

Overall RNA removal was 99.84% (See table 9 below).

**Table 9**

| | **Vol (ml)** | **RNA conc (□g/ml)** | **Step removal** | **Overall removal** |
|---|---|---|---|---|
| **Lysate** | 1800 | 1579.1 | NA | NA |
| UF1 conc permeate | 1700 | 123.2 | 7.4% | NA |
| UF1 diafiltration permeate | 5000 | 354.8 | 62.0% | NA |
| **UF1 retentate** | 280 | 2552.4 | 74.9% | 74.9% |
| **UF1 post CaCl₂ pption** | 480 | 6.25 | 99.44% | 99.86% |
| TMAE peak 1 | 240 | 18.21 | >100% | NA |
| **TMAE peak 2** | 75 | 21.95 | 0% | 99.84% |

The improved UF1 step resulted in 74.9% RNA clearance. The fact that most of the RNA was cleared during diafiltration and that the remaining RNA was essentially of high molecular weight suggests that the RNA initially participated in the gel layer but was then "resolubilised" by the low ionic strength diafiltration buffer and allowed to pass through the membrane due to its small size. 99.44% of the RNA recovered in the UF1 retentate was then precipitated by treatment with calcium chloride. It is well known that calcium chloride is a particularly potent precipitant of high molecular weight RNA. The anion exchange column however did not provide further RNA removal although RNA was detected in the column breakthrough: there appears to be a contradiction in the RNA assay results.

Analysis by size exclusion HPLC shows the effect of treatment with calcium chloride: the plasmid/RNA ratio rose from 16.11% in the UF1 retentate to 89.76% after precipitation with calcium chloride (See table 10 below)

**Table 10**

| | **%plasmid** | **%RNA** |
|---|---|---|
| UF1 retentate | 16.11 % | 83.89% |
| UF1 post CaCl₂ pption | 89.76% | 10.24% |
| TMAE peak 1 | 0% | 100.0% |
| TMAE peak 2 | 98.95% | 1.05% |

At the end of the process, the RNA content was only 1.05%.

Analysis of plasmid topology by ion exchange-HPLC shows that the process did not affect the levels of plasmid isoforms: throughout the process about 96% of plasmids were supercoiled with about 3.4% multimeric (See table 11 below)

**Table 11**

| | **%open circle** | **%supercoiled** | **%multimers** |
|---|---|---|---|
| UF1 retentate | 0.77% | 95.76% | 3.47% |
| UF1 post CaCl₂ pption | 0% | 96.69% | 3.31 % |
| TMAE peak 2 | 0% | 96.57% | 3.43% |

The UF1 step removed 99.87% of proteins (See table 12 below)

**Table 12**

| | **Vol (ml)** | **protein conc (□g/ml)** | **Step removal** | **Overall removal** |
|---|---|---|---|---|
| **Lysate** | 1800 | 5768.6 | NA | NA |
| UF1 conc permeate | 1700 | 5565.5 | 91.1% | NA |
| UF1 diafiltration permeate | 5000 | 277.8 | 13.3% | NA |
| **UF1 retentate** | 280 | 52.6 | 99.87% | 99.87% |
| **UF1 post CaCl₂ pption** | 480 | ND* | ND | ND |
| TMAE peak 1 | 240 | ND | ND | ND |
| **TMAE peak 2** | 75 | 0 | 100.0% | 100.0% |

| | | | | |
|---|---|---|---|---|
| * a cloudy precipitate formed due to the presence of calcium chloride salt | | | | |

Proteins are small enough to be efficiently cleared through a 300K membrane. At the end of the process, no protein could be detected in the Fractogel TMAE eluate.

Overall endotoxin removal was high at more than 99.94% (See table 13 below)

**Table 13**

| | **Vol (ml)** | **endotoxin conc (EU/ml)** | **Step removal** | **Overall removal** |
|---|---|---|---|---|
| **Lysate** | 1800 | 102.0 | NA | NA |
| UF1 conc permeate | 1700 | <5.0 | <4.6% | NA |
| UF1 diafiltration permeate | 5000 | 44.0 | 119.8% | NA |
| **UF1 retentate** | 280 | 540.5 | 17.6% | 17.6% |
| **UF1 post CaCl₂ pption** | 480 | <25.0 | >89.4% | >91.3% |
| TMAE peak 1 | 240 | 0.65 | NA | NA |
| **TMAE peak 2** | 75 | <0.50 | NA | >99.94% |

The calcium chloride step gave high endotoxin removal: more than 89.4%. The anion exchange step also provided endotoxin clearance although the exact figure could not be calculated. The UF1 step did not have a significant effect on endotoxin levels: only 17.6% was cleared at this stage.

Genomic DNA levels were reduced by a combination of steps: 55% reduction for the UF1 step, 77.6% for the calcium chloride step and 66.1% for the anion exchange step (See table 14 below which shows the effect of the nucleic acid concentration in the load on the performance of UF1 without recirculation in the presence of calcium chloride)

**Table 14**

| **UF1 load (mg plasmid/ft²)** | **Plasmid conc (µg/ml)** | **Plasmid recovery** | | | **RNA content (SEC-HPLC)** |
|---|---|---|---|---|---|
| | | **Conc permeate** | **Diaf permeate** | **UF1 retentate** | |
| 126.5* | 69.9 | 0.2% | 0 | 108.8% | 4.64% |
| 109.3** | 60.7 | 1.3% | 0 | 90.5% | 0 |
| 129.6** | 36.0 | 5.9% | 7.2% | 82.7% | 1.93% |

| | | | | | |
|---|---|---|---|---|---|
| plasmid construct: *7.7kbp, **5.9kbp | | | | | |

The overall clearance was 96.6%. The calcium chloride step did not have the impact expected probably due to the low concentration of 0.3M used.

### Process option selection: option 3

Option 3 was very similar to 2 except that a second UF step was introduced to remove calcium chloride salts before loading on to the anion exchange column. The membrane used for the UF2 step had a lower molecular weight cut-off of 100K to prevent losses of plasmid in the presence of calcium chloride salt. Low ionic strength buffer was used at the beginning of diafiltration to promote the clearance of RNA followed by 50mM Tris + 0.54M NaCl to buffer exchange before loading on to the chromatography column. A low initial TMP of 5psi was selected to improve plasmid recovery. The membrane load was 909mg of plasmid per square foot of membrane.

Most of the remaining RNA was cleared during concentration and early diafiltration. The anion exchange step was carried out as for option 2

Plasmid recovery across the UF1 step was 74.5%, then over 100% across the calcium chloride precipitation step and 71.1 % for the UF2 step (See table 15 below).

**Table 15**

| | **Vol (ml)** | **Plasmid conc (□g/ml)** | **Step recovery** | **Overall recovery** |
|---|---|---|---|---|
| **Lysate** | 1800 | 71.2 | NA | NA |
| UF1 conc permeate | 1730 | 0 | 0 | NA |
| UF1 diafiltration permeate | 5000 | 0 | 0 | NA |
| **UF1 retentate** | 315 | 303.0 | 74.5% | 74.5% |
| **UF1 post CaCl₂ pption** | 330 | 284.3 | 105.0% | 78.2% |
| UF2 conc permeate | 260 | 2.0 | 0.6% | NA |
| UF2 diafiltration permeate | 1000 | 0.77 | 0.8% | NA |
| **UF2 retentate** | 105 | 635.2 | 71.1% | 57.3% |
| TMAE peak 1 | 105 | 201.6 | 37.0% | 21.2% |
| **TMAE peak 2** | 90 | 305.8 | 41.3% | 27.6% |

A peak of absorbance was detected in the breakthrough of the Fractogel TMAE column: 37% of the plasmid was lost in the breakthrough with 41.3% recovered in the eluate. The overall recovery of the combined fractions was 48.8%. Although the column load was 2.86mg of plasmid per ml of column, which is slightly higher than in option 2 it is doubtful this can account for the heavy plasmid losses observed. The load buffer was also different: the column was loaded in 0.15M calcium chloride and 0.1M Tris in option 2 compared with 50mM Tris + 0.54M NaCl for option 3, so the ionic strength of the loading buffer may have been too high.

Overall RNA removal was very high at 99.985% (See table 16 below)

**Table 16**

| | **Vol (ml)** | **RNA conc (µg/ml)** | **Step removal** | **Overall removal** |
|---|---|---|---|---|
| **Lysate** | 1800 | 1749.5 | NA | NA |
| UF1, conc permeate | 1730 | 117.2 | 6.4% | NA |
| UF1 diafiltration permeate | 5000 | 357.0 | 56.7% | NA |
| **UF1 retentate** | 315 | 2281.9 | 77.2% | 77.2% |
| **UF1 post CaCl₂ pption** | 330 | 25.0 | 98.8% | 99.72% |
| UF2 conc permeate | 260 | 185.4 | >100.0% | NA |
| UF2 diafiltration permeate | 1000 | 10.74 | >100.0% | NA |
| **UF2 retentate** | 105 | 19.12 | 74.9% | 99.93% |
| TMAE peak 1 | 105 | 5.84 | 35.6% | NA |
| **TMAE peak 2** | 90 | 4.20 | 78.0% | 99.985% |

The RNA clearance was 77.2% for the UF1 step, 98.8% for the calcium chloride precipitation step, 74.9% for the UF2 step and 78% for the chromatography column. These results are very similar to those obtained in process 2 except for the added clearance provided by the UF2 step.

Analysis by size exclusion-HPLC shows an increase in the plasmid/RNA ratio from 17.02% in the UF1 retentate to 92.25% after precipitation with calcium chloride, 95.24% in the UF2 retentate and 98.91% after the anion exchange chromatography step (See table 17).

**Table 17**

| | **%plasmid** | **%RNA** |
|---|---|---|
| UF1 retentate | 17.02% | 82.98% |
| UF1 post CaCl₂ pption | 92.25% | 7.75% |
| UF2 conc permeate | 22.67% | 77.33% |
| UF2 diafiltration permeate | 4.93% | 95.07% |
| UF2 retentate | 95.24% | 4.76% |
| TMAE peak 1 | 92.01% | 7.99% |
| TMAE peak 2 | 98.91% | 1.09% |

Analysis of plasmid topology by ion exchange-HPLC shows that about 96% of plasmids are supercoiled and 3.5% multimeric at the UF1 retentate stage and after precipitation with calcium chloride (See table 18 below).

**Table 18**

| | **%open circle** | **%supercoiled** | **%multimers** |
|---|---|---|---|
| UF1 retentate | 0% | 96.21% | 3.79% |
| UF1 post CaCl₂ pption | 0% | 96.49% | 3.51% |
| UF2 retentate | 22.73% | 75.70% | 1.57% |
| TMAE peak 2 | 20.93% | 78.54% | 0.53% |

After the UF2 step, 22.73% of plasmids are open circular with only 75.5% supercoiled. It is possible that the pump on the Labscale UF system used for the UF2 step was unsuitable and sheared the plasmid resulting in high levels of open circle. From previous experience at this stage, it is not believed that the UF2 step in itself is unsuitable. This finding however seems to explain the high plasmid levels in the breakthrough of the chromatography step: it is quite likely that the wash buffer of 50mM Tris + 0.54M NaCl was designed to elute open circular plasmids. In this buffer, open circular plasmid loaded on to the column would then be prevented from binding.

Protein clearance at the UF1 stage was 99.86% resulting in an overall clearance of 99.997% (See table 19 below).

**Table 19**

| | **Vol (ml)** | **Protein conc (□g/ml)** | **Step removal** | **Overall removal** |
|---|---|---|---|---|
| **Lysate** | 1800 | 5881.5 | NA | NA |
| UF1 conc permeate | 1730 | 5373.6 | 87.8% | NA |
| UF1 diafiltration permeate | 5000 | 277.8 | 13.1% | NA |
| **UF1 retentate** | 315 | 45.9 | 99.86% | 99.86% |
| **UF1 post CaCl₂ pption** | 330 | ND* | ND | ND |
| **UF2 retentate** | 105 | 3.7 | ND | 99.996% |
| **TMAE peak 2** | 90 | 2.94 | 20.5% | 99.997% |

| | | | | |
|---|---|---|---|---|
| *a cloudy precipitate formed due to the presence of calcium chloride salt | | | | |

Endotoxin removal was only 27.9% at the UF1 stage but was 90% across the calcium chloride precipitation step (See table 20 below).

**Table 20**

| | **Vol (ml)** | **Endotoxin cone (EU/ml)** | **Step removal** | **Overall removal** |
|---|---|---|---|---|
| **Lysate** | 1800 | 95.40 | NA | NA |
| UF1 conc permeate | 1730 | <5.0 | <5.0% | NA |
| UF1 diafiltration permeate | 5000 | 17.53 | 51.0% | NA |
| **UF1 retentate** | 315 | 392.9 | 27.9% | 27.9% |
| **UF1 post CaCl₂ pption** | 330 | 35.07 | 90.0% | 92.8% |
| UF2 conc permeate | 260 | <5.0 | <11.2% | NA |
| UF2 diafiltration permeate | 1000 | 0.15 | 1.3% | NA |
| **UF2 retentate** | 105 | 69.62 | 36.8% | 95.3% |
| TMAE peak 1 | 105 | 1.96 | 3.3% | NA |
| **TMAE peak 2** | 90 | <0.5 | >99.3% | >99.967% |

The UF2 step also gave a low clearance of 36.8% confirming that ultrafiltration is not a significant endotoxin removal step. The chromatography step on Fractogel TMAE however removed more than 99.3% of the remaining endotoxin bringing the overall clearance to more than 99.97%.

Genomic DNA removal was essentially the result of the calcium chloride precipitation step: 94% clearance was observed (See table 21).

**Table 21**

| | **Vol (ml)** | **gDNA cone (µg/ml)** | **Step removal** | **Overall removal** |
|---|---|---|---|---|
| **Lysate** | 1800 | 1.74 | NA | NA |
| UF1 conc permeate | 1730 | 0.01 | 0.6% | NA |
| UF1 diafiltration permeate | 5000 | 0.14 | 22.3% | NA |
| **UF1 retentate** | 315 | 12.64 | 0% | 0% |
| **UF1 post CaCl₂ pption** | 330 | 0.68 | 94.0% | 92.4% |
| UF2 conc permeate | 260 | 0.01 | 1.2% | NA |
| UF2 diafiltration permeate | 1000 | 0.004 | 1.8% | NA |
| **UF2 retentate** | 105 | 0.57 | 72.5% | 97.9% |
| TMAE peak 1 | 105 | 0.38 | 77.8% | NA |
| **TMAE peak 2** | 90 | 0.23 | 59.6% | 99.2% |

The other step removal figures were: 0% for the UF1 step, 72.5% for the UF2 step and 59.6% for the chromatography step. The overall genomic DNA removal was 99.2%

### Process option selection: option 4

The cell debris removal step post alkaline lysis was combined with the calcium chloride precipitate removal step. Calcium chloride stock solution at 2M was added to the lysate after neutralisation with potassium acetate so that the final calcium chloride concentration was 1M. Cell debris was then removed by a sieve filter with 200µm pores then smaller particulates were filtered out on a depth filter. The UF1 step was carried out on a 100K, 1ft² PES membrane to prevent plasmid losses in the presence of calcium chloride salt. A low ionic strength buffer was used at the start of diafiltration to maximise RNA clearance followed by 50mM Tris + 0.54M NaCl to buffer exchange before loading on to the anion exchange column. The starting TMP was reduced to 5psi to improve plasmid recovery. Membrane load was 240mg of plasmid per square foot of membrane. Anion exchange chromatography on Fractogel TMAE was conducted as before loading at 2.83mg of plasmid per ml of column.

Plasmid recovery across the calcium chloride step was more than 100%, then 50.8% across the UF1 step (See table 22).

**Table 22**

| | **Vol (ml)** | **Plasmid conc (□g/ml)** | **Step recovery** | **Overall recovery** |
|---|---|---|---|---|
| **Lysate** | 1800 | 97.5 | NA | NA |
| **Lysate post CaCl₂ pption*** | 3350 | 71.8 | 137.0% | 137.0% |
| UF1 conc permeate* | 3350 | 0 | 0 | NA |
| UF1 diafiltration permeate* | 5000 | 0.16 | 0.3% | NA |
| **UF1 retentate** | 290 | 452.4 | 50.8% | 74.8% |
| TMAE peak 1 | 135 | 66.8 | 15.9% | 11.9% |
| **TMAE peak 2** | 135 | 341.4 | 81.5% | 60.9% |

| | | | | |
|---|---|---|---|---|
| * desalted sample | | | | |

15.9% of the plasmid loaded was lost in the breakthrough while 81.5% was eluted: the column may have been overloaded at 2.83mg/ml. The overall plasmid recovery of the 2 fractions combined was 72.8%.

Overall RNA clearance was 99.9% (See table 23).

**Table 23**

| | **Vol (ml)** | **RNA conc (□g/ml)** | **Step removal** | **Overall removal** |
|---|---|---|---|---|
| **Lysate** | 1800 | 1968.5 | NA | NA |
| **Lysate post CaCl₂ pption*** | 3350 | 162.6 | 84.6% | 84.6% |
| UF1 conc permeate* | 3350 | 126.7 | 77.9% | NA |
| UF1 diafiltration permeate* | 5000 | 84.5 | 77.6% | NA |
| **UF1 retentate** | 290 | 42.7 | 97.7% | 99.65% |
| TMAE peak 1 | 135 | 8.60 | 21.8% | NA |
| **TMAE peak 2** | 135 | 10.98 | 72.2% | 99.90% |

| | | | | |
|---|---|---|---|---|
| *desalted samples | | | | |

The calcium chloride precipitation step accounted for 84.6% of the RNA removed, essentially high molecular weight RNA. The low molecular weight RNA was then easily cleared by the UF1 step (97.7% removal). RNA clearance across the chromatography column was 72.2%.

Analysis by size exclusion HPLC showed a plasmid level of 97.7% in the UF1 retentate, increasing to 98.62% in the Fractogel TMAE eluate (See table 24).

**Table 24**

| | **%plasmid** | **%RNA** |
|---|---|---|
| UF1 retentate | 97.70% | 2.30% |
| TMAE peak 1 | 97.07% | 2.93% |
| TMAE peak 2 | 98.62% | 1.38% |

Analysis of plasmid topology by ion exchange-HPLC showed that the lysate post calcium chloride precipitation consisted of 84.44% supercoiled plasmid and 14% open circular (See table 25).

**Table 25**

| | **%open circle** | **%supercoiled** | **%multimers** |
|---|---|---|---|
| Lysate post CaCl₂ pption | 14.0% | 84.44% | 1.56% |
| UF1 retentate | 3.62% | 93.34% | 3.04% |
| TMAE peak 2 | 2.16% | 94.76% | 3.08% |

The proportion of supercoiled increased to 94.76% in the Fractogel TMAE eluate with 2.16% open circle and 3.08% multimers.

Protein could not be detected in the Fractogel TMAE eluate. The calcium chloride precipitation step removed 95.8% of the protein while the UF1 step cleared 99.7% of the remainder (See table 26 below).

**Table 26**

| | **Vol (ml)** | **Protein conc (µg/ml)** | **Step removal** | **Overall removal** |
|---|---|---|---|---|
| **Lysate** | 1800 | 6107.2 | NA | NA |
| **Lysate post CaCl₂ pption*** | 3350 | 138.6 | 95.8% | 95.8% |
| UF1 conc permeate* | 3350 | 117.4 | 84.7% | NA |
| UF1 diafiltration permeate* | 5000 | 0.52 | 0.6% | NA |
| UF1 retentate | 290 | 5.2 | 99.7% | 99.99% |
| **TMAE peak 2** | 135 | 0 | 100.0% | 100.0% |

| | | | | |
|---|---|---|---|---|
| *desalted samples | | | | |

The overall removal of endotoxin was more than 99.5% (See table 27).

**Table 27**

| | **Vol (ml)** | **Endotoxin conc (EU/ml)** | **Step removal** | **Overall removal** |
|---|---|---|---|---|
| **Lysate** | 1800 | 18.84 | NA | NA |
| **Lysate post CaCl₂ pption*** | **3350** | <11.05 | >0% | >0% |
| UF1 conc permeate* | 3350 | <11.05 | <100.0% | NA |
| UF1 diafiltration permeate* | 5000 | <1.1 | <14.9% | NA |
| **UF1 retentate** | 290 | <50.0 | >60.8% | >57.2% |
| TMAE peak 1 | 135 | 2.43 | <5.2% | NA |
| **TMAE peak 2** | 135 | <0.5 | >98.9% | >99.5% |

| | | | | |
|---|---|---|---|---|
| *desalted sample | | | | |

It was not possible to determine which steps contributed to the removal from the assay results although clearance on the Fractogel TMAE column was more than 98.9%.

Overall genomic DNA removal was 99.8% (See table 28 below)

**Table 28**

| | **Vol (ml)** | **gDNA conc (µg/ml)** | **Step removal** | **Overall removal** |
|---|---|---|---|---|
| **Lysate** | 1800 | 2.11 | NA | NA |
| **Lysate post CaCl₂ pption*** | 3350 | 0.02 | 98.0% | 98.0% |
| UF1 conc permeate* | 3350 | 0.002 | 0.2% | NA |
| UF1 diafiltration permeate* | 5000 | 0.005 | 0.7% | NA |
| **UF1 retentate** | 290 | 0.006 | 74.0% | 99.5% |
| TMAE peak 1 | 135 | 0.02 | 16.7% | NA |
| **TMAE peak 2** | 135 | 0.03 | 75.0% | 99.8% |

| | | | | |
|---|---|---|---|---|
| * desalted sample | | | | |

98% of the genomic DNA was removed at the calcium chloride precipitation stage, 74% by the UF1 step and 75% by the chromatography step.

The benefits of the alternative processes can be most simply compared in table 29 below.

**Table 29**

| | **Option 2** | **Option 3** | **Option 4** | **Product spec** |
|---|---|---|---|---|
| Plasmid recovery | 89.2% | 48.8%* | 72.8%* | |
| RNA removal (RP-HPLC) | 99.84% | 99.985% | 99-90% | |
| RNA w/w ratio | 3.65% | 1.37% | 3.13% | <5% |
| %plasmid (SEC-HPLC) | 98.95% | 98.,91% | 98.62% | |
| Plasmid topology (%supercoiled) | 96.57% | 78.54% | 94.76% | |
| Protein removal | 100.0% | 99.997% | 100.0% | |
| Protein w/w ratio | 0% | 0.96% | 0% | <1% |
| Endotoxin removal | >99.94% | >99.967% | >99.54% | |
| Endotoxin EU/mg | <0.83EU/ mg | <1.63EU/m g | <1.54EU/m g | <100EU/m g |
| gDNA removal | 96.6% | 99.2% | 99.8% | |
| gDNA w/w ratio | 0.45% | 0.07% | 0.02% | <5% |

| | | | | |
|---|---|---|---|---|
| *peak1+peak2 | | | | |

Overall plasmid recovery was higher with options 2 (89.2%) and 4 (72.8% for the combined peaks) than option 3 (48.8% for the combined peaks). No single step gave low plasmid recoveries. The low overall plasmid recovery for option 3 is essentially due to the additional UF step introduced.

RNA removal was high with all 3 options (99.84-99.985%). Option 3 resulted in an RNA content of 1.37% weight per weight of plasmid. However options 2 and 4 gave RNA contents of 3.65 and 3.13% respectively.Both the ultrafiltration and the calcium chloride steps performed efficiently, in fact combining well with the ultrafiltration step removing low molecular weight RNA and calcium chloride precipitating high molecular weight RNA. Options 3 and 4 gave suitable levels of supercoiled plasmid at 96.6 and 94.8% respectively. Option 3 however generated high proportions of open circular plasmid resulting in only 78.5% supercoiled plasmid. This was due to the ultrafiltration system used for UF2 and should not eliminate the option.

Protein removal was high for all 3 options with undetectable levels for options 3 and 4. A protein ratio of 0.96% weight per weight for option 3 may be due to the tendency of the BCA assay to generate false results due to contamination rather than accurate representation of protein levels There is no reason to expect higher protein levels in option 3 where there is one additional purification step compared with option 2. Both the UF1 step and the calcium chloride step are very efficient at removing protein impurities.

Endotoxin removal was very high with levels below the detection limit of the assay in the final material for all 3 options.

The calcium chloride precipitation step and the anion exchange step both gave high endotoxin clearance.

Finally, genomic DNA levels were very low especially for options 3 and 4. High genomic DNA removal seemed to be linked to high calcium chloride levels.

After evaluating all the data carefully, there seemed to be no reason to eliminate any of the 3 options on the basis of impurity removal since all 3 options gave good removal of impurities. Option 3 gave the lowest plasmid recovery but also the best RNA clearance. Option 4 was preferred because it contains fewer purification steps and would be the easiest option to scale up.

### CONCLUSION

The three options differed only in where the calcium chloride precipitation step was introduced (either after the lysis or after the UF1 step) and whether a second ultrafiltration step was required to remove calcium chloride salt before chromatography. Results showed that all 3 options were viable giving more than 50% plasmid recovery and good impurity removal.

RNA removal was improved by loading the chromatography column at a reduced level of 1.2mg plasmid/ml and using a gradient elution. In fact, with the right elution gradient and peak cutting, final RNA levels were so low as to be below the detection limit of the assay.

Calcium chloride had a very significant effect in reducing the levels of RNA, protein, endotoxin and genomic DNA. As a result, final levels of endotoxin and genomic DNA are lower than in the process containing RNase.

The application of which this description and claims form a part may be used as a basis for priority in respect of any subsequent application. The claims of such subsequent application may be directed to any feature or combination of features described herein. They may take the form of product, composition, process or use claims and may include, by way of example and without limitation, one or more of the following claims:

## Claims

1. A method of separating extra-chromosomal DNA from RNA without first digesting the RNA, said method comprising:
i) lysing cells comprising extra-chromosomal DNA to form a lysate; and
ii) separating a substantial amount of the RNA present in the lysate from the extra chromosomal DNA using a combination of calcium chloride precipitation, clarification and tangential flow ultrafiltration.

2. A method as claimed in claim1 wherein the DNA obtained comprises less than 50 % by weight of the RNA present in the lysate.

3. A method as claimed in claim 2 wherein the DNA obtained comprises less than 10 % by weight of the RNA present in the lysate.

4. A method as claimed in claim 3 wherein the DNA obtained comprises less than 5 % by weight of the RNA present in the lysate.

5. A method as claimed in claim 4 wherein the DNA obtained comprises less than 2 % by weight of the RNA present in the lysate.

6. A method as claimed in claim 5 wherein the DNA obtained comprises less than 1 % by weight of the RNA present in the lysate.

7. A method as claimed in any of claims 1 to 6 wherein the calcium chloride precipitation step precedes the ultra filtration step.

8. A method as claimed in any of claim 1 to 6 wherein the calcium chloride precipitation step follows the ultra filtration step.

9. A method as claimed in any of claims 1 to 8 wherein one or more additional purification or polishing steps are used.

10. A method as claimed in claim 9 wherein the one or more additional purification or polishing steps are to remove one or more of the following entities: endotoxins; proteins; genomic DNA and RNA.

11. A method as claimed in claim 9 or 10 wherein the one or more additional purification or polishing steps follow the ultra filtration step.

12. A method as claimed in any of claims 9, 10 or 11 wherein the one or more additional purification or polishing steps comprises a chromatography step.

13. A method as claimed in claim 12 wherein the chromatography step is an ion exchange or hydroxyapatite chromatography step.

14. A method as claimed in claim 13 wherein the chromatography step is an ion exchange chromatography step.

15. A method as claimed in claim 14 wherein the ion exchange chromatography step utilises Fractogel TMAE.

16. A method as claimed in any of the preceding claims wherein a precipitate formed in the calcium chloride precipitate step is removed by a mesh or sieve.

17. A method as claimed in claim 16 wherein a precipitate formed in the calcium chloride precipitate step is removed along with cell debris by a mesh or sieve.

18. A method as claimed in any of the preceding claims wherein the CaCl₂ precipitation step removes high molecular weight RNA and genomic DNA.

19. A method as claimed in any of claims 1-18 wherein the ultrafiltration step utilises a membrane with a molecular weight cut off of below 500 KDa and the extra-chromosomal DNA is of from 2 Kb to 200 Kb.

20. A method as claimed in claim 19 wherein the membrane has a molecular weight cut off of below 300 KDa, more preferably about 100 KDa.

21. A method as claimed in claim 19 wherein the membrane is selected from the group consisting of: polyethersulphone; cellulose acetate; polysulphone; polyvinylidene; polypropylene; polyamide; modified PES; polyvinylidine pyrrolidine; polyvinylidine fluoride (PVDF); cellulose nitrate and cellulose triacetate.

22. A method as claimed in claim 21 wherein the membrane is a polyethersulphone membrane.

23. A method as claimed in any of the preceding claims which utilises a diafiltration buffer having a pH range of from 6-10.

24. A method as claimed in claim 23 wherein the diafiltration buffer is a Tris buffer.

25. A method as claimed in claim 23 wherein the diafiltration buffer is used in greater than 10 volume exchanges.

26. A method as claimed in claim 25 wherein the diafiltration buffer is used in greater than 20 volume exchanges.

27. A method as claimed in claim 26 wherein the diafiltration buffer is used in greater than 30 volume exchanges.

28. A method as claimed in claim 27 wherein the diafiltration buffer is used in greater than 40 volume exchanges.

29. A method as claimed in claim 28 wherein the diafiltration buffer is used in from 50 to 100 volume exchanges.

30. A method as claimed in claim 19 wherein the membrane is used with a screen channel.

31. A method as claimed in claim 30 wherein the screen channel is a T screen.

32. A method as claimed in any of the preceding claims wherein the method is conducted at a transmembrane pressure of less than 10psi.

33. A method as claimed in claim 19 wherein the extrachomosomal DNA is loaded onto the membrane in an amount of less than 30mg/ft2 in the case of a 300K membrane.

34. A method as claimed in claim 13 wherein the chromatography step is a hydroxyapatite chromatography step.

35. A method as claimed in claim 34 wherein the hydroxyapatite chromatography step is conducted at a pH of from 7.3 to 8.3.

36. A method as claimed in claim 35 wherein the hydroxyapatite chromatography step is conducted at a pH of between 7.6 and 8.0.

37. A method as claimed in claim 34, 35 or 36 wherein the hydroxyapatite is BioRad MacroPrep Ceramic.

38. A method as claimed in any of claims 34 to 37 wherein a double buffer system is used to separate extra chromosomal DNA from RNA.

39. A method as claimed in claim 38 wherein the double buffer system comprises Na₂HPO₄ to elute the RNA and K₂HPO₄ to elute the extra chromosomal DNA.

40. A method as claimed in claim 8 wherein the CaCl₂ precipitation step is followed by a further ultra filtration step or a desalting step or a reverse phase chromatography step.

41. A method as claimed in claim 40 wherein the further ultra filtration step or a desalting step or a reverse phase chromatography step is followed by the chromatography step.

42. A method as claimed in claim 41 wherein the chromatography step is an ion exchange.

## Patentansprüche

1. Verfahren zum Trennen extra-chromosomaler DNA von RNA, ohne die RNA zuerst zu verdauen, wobei das Verfahren folgende Schritte umfaßt:
i) Lysieren von Zellen, die extra-chromosomale DNA umfassen, um ein Lysat zu bilden; und
ii) Trennen einer substantiellen Menge der RNA, die in dem Lysat vorliegt, von der extra-chromosomalen DNA unter Verwendung einer Kombination aus Calciumchlorid-Präzipitation, Klärung und Tangentialfluß-Ultrafiltration.

2. Verfahren gemäß Anspruch 1, worin die erhaltene DNA weniger als 50 Gew.% der RNA, die in dem Lysat vorliegt, umfaßt.

3. Verfahren gemäß Anspruch 2, worin die erhaltene DNA weniger als 10 Gew.% der RNA, die in dem Lysat vorliegt, umfaßt.

4. Verfahren gemäß Anspruch 3, worin die erhaltene DNA weniger als 5 Gew.% der RNA, die in dem Lysat vorliegt, umfaßt.

5. Verfahren gemäß Anspruch 4, worin die erhaltene DNA weniger als 2 Gew.% der RNA, die in dem Lysat vorliegt, umfaßt.

6. Verfahren gemäß Anspruch 5, worin die erhaltene DNA weniger als 1 Gew.% der RNA, die in dem Lysat vorliegt, umfaßt.

7. Verfahren gemäß einem der Ansprüche 1 bis 6, worin der Calciumchlorid-Präzipitationsschritt dem Ultrafiltrationsschritt vorangeht.

8. Verfahren gemäß einem der Ansprüche 1 bis 6, worin der Calciumchlorid-Präzipitationsschritt dem Ultrafiltrationsschritt folgt.

9. Verfahren gemäß einem der Ansprüche 1 bis 8, worin ein oder mehrere zusätzliche Aufreinigungs- oder Polierschritte verwendet werden.

10. Verfahren gemäß Anspruch 9, worin der eine oder die mehreren zusätzlichen Aufreinigungs- oder Polierschritte verwendet werden, um eine oder mehrere der folgenden Einheiten zu entfernen: Endotoxine, Proteine, genomische DNA und RNA.

11. Verfahren gemäß Anspruch 9 oder 10, worin der eine oder die mehreren zusätzlichen Aufreinigungs- oder Polierschritte dem Ultrafiltrationsschritt folgen.

12. Verfahren gemäß Anspruch 9, 10 oder 11, worin der eine oder die mehreren zusätzlichen Aufreinigungs- oder Polierschritte einen Chromatographieschritt umfassen.

13. Verfahren gemäß Anspruch 12, worin der Chromatographieschritt ein Ionenaustausch- oder Hydroxylapatit-Chromatographieschritt ist.

14. Verfahren gemäß Anspruch 13, worin der Chromatographieschritt ein Ionenaustausch-Chromatographieschritt ist.

15. Verfahren gemäß Anspruch 14, worin der Ionenaustausch-Chromatographieschritt Fractogel TMAE verwendet.

16. Verfahren gemäß einem der vorangegangenen Ansprüche, worin ein Präzipitat, das in dem Calciumchlorid-Präzipitationsschritt gebildet wird, durch ein Netz oder Sieb entfernt wird.

17. Verfahren gemäß Anspruch 16, worin ein Präzipitat, das in dem Calciumchlorid-Präzipitationsschritt gebildet wird, zusammen mit Zelltrümmern durch ein Netz oder Sieb entfernt wird.

18. Verfahren gemäß einem der vorangegangenen Ansprüche, worin der CaCl₂-Präzipitationsschritt RNA mit hohem Molekulargewicht und genomische DNA entfernt.

19. Verfahren gemäß einem der Ansprüche 1 bis 18, worin der Ultrafiltrationsschritt eine Membran mit einem Molekulargewichtsausschluß von unterhalb 500 KDa verwendet und die extra-chromosomale DNA von 2 Kb bis 200 Kb ist.

20. Verfahren gemäß Anspruch 19, worin die Membran einen Molekulargewichtsausschluß von unterhalb 300 KDa, besonders bevorzugt von ungefähr 100 KDa, hat.

21. Verfahren gemäß Anspruch 19, worin die Membran aus der Gruppe ausgewählt ist, die aus folgendem besteht: Polyethersulfon, Celluloseacetat, Polysulfon, Polyvinyliden, Polypropylen, Polyamid, modifiziertes PES, Polyvinylidenpyrrolidin, Polyvinylidenfluorid (PVDF), Cellulosenitrat und Cellulosetriacetat.

22. Verfahren gemäß Anspruch 21, worin die Membran eine Polyethersulfonmembran ist.

23. Verfahren gemäß einem der vorangegangenen Ansprüche, das einen Diafiltrationspuffer verwendet, der einen pH-Bereich von 6 bis 10 hat.

24. Verfahren gemäß Anspruch 23, worin der Diafiltrationspuffer ein Tris-Puffer ist.

25. Verfahren gemäß Anspruch 23, worin der Diafiltrationspuffer mit mehr als 10 Volumenaustauschungen verwendet wird.

26. Verfahren gemäß Anspruch 25, worin der Diafiltrationspuffer mit mehr als 20 Volumenaustauschungen verwendet wird.

27. Verfahren gemäß Anspruch 26, worin der Diafiltrationspuffer mit mehr als 30 Volumenaustauschungen verwendet wird.

28. Verfahren gemäß Anspruch 27, worin der Diafiltrationspuffer mit mehr als 40 Volumenaustauschungen verwendet wird.

29. Verfahren gemäß Anspruch 28, worin der Diafiltrationspuffer mit 50 bis 100 Volumenaustauschungen verwendet wird.

30. Verfahren gemäß Anspruch 19, worin die Membran mit einem Screen Channel verwendet wird.

31. Verfahren gemäß Anspruch 30, worin der Screen Channel ein T-Screen ist.

32. Verfahren gemäß einem der vorangegangenen Ansprüche, worin das Verfahren bei einem Transmembrandruck von weniger als 10 psi durchgeführt wird.

33. Verfahren gemäß Anspruch 19, worin die extra-chromosomale DNA auf die Membran in einer Menge von weniger als 30 mg/ft² im Fall einer 300 K-Membran geladen wird.

34. Verfahren gemäß Anspruch 13, worin der Chromatographieschritt ein Hydroxylapatit-Chromatographieschritt ist.

35. Verfahren gemäß Anspruch 34, worin der Hydroxylapatit-Chromatographieschritt bei einem pH von 7,3 bis 8,3 durchgeführt wird.

36. Verfahren gemäß Anspruch 35, worin der Hydroxylapatit-Chromatographieschritt bei einem pH zwischen 7,6 und 8,0 durchgeführt wird.

37. Verfahren gemäß Anspruch 34, 35 oder 36, worin der Hydroxylapatit BioRad MacroPrep Ceramic ist.

38. Verfahren gemäß einem der Ansprüche 34 bis 37, worin ein Doppelpuffersystem zum Trennen extra-chromosomaler DNA von RNA verwendet wird.

39. Verfahren gemäß Anspruch 38, worin das Doppelpuffersystem Na₂HPO₄, um die RNA zu eluieren, und K₂HPO₄, um die extra-chromosomale DNA zu eluieren, umfaßt.

40. Verfahren gemäß Anspruch 8, worin der CaCl₂-Präzipitationsschritt von einem weiteren Ultrafiltrationsschritt oder einem Entsalzungsschritt oder einem Umkehrphasen-Chromatographieschritt gefolgt wird.

41. Verfahren gemäß Anspruch 40, worin der weitere Ultrafiltrationsschritt oder ein Entsalzungsschritt oder ein Umkehrphasen-Chromatographieschritt vom Chromatographieschritt gefolgt wird.

42. Verfahren gemäß Anspruch 41, worin der Chromatographieschritt ein Ionenaustausch-Chromatographieschritt ist.

## Revendications

1. Procédé pour séparer l'ADN extrachromosomique de l'ARN sans digestion préalable de l'ARN, ledit procédé comprenant :
i) lyser des cellules comprenant de l'ADN extrachromosomique pour former un lysat; et
ii) séparer une quantité substantielle de l'ARN présent dans le lysat de l'ADN extrachromosomique en utilisant une combinaison de précipitation au chlorure de calcium, clarification et ultrafiltration à flux tangentiel.

2. Procédé tel que revendiqué dans la revendication 1, dans lequel l'ADN obtenu comprend moins de 50 % en poids de l'ARN présent dans le lysat.

3. Procédé tel que revendiqué dans la revendication 2, dans lequel l'ADN obtenu comprend moins de 10 % en poids de l'ARN présent dans le lysat.

4. Procédé tel que revendiqué dans la revendication 3, dans lequel l'ADN obtenu comprend moins de 5 % en poids de l'ARN présent dans le lysat.

5. Procédé tel que revendiqué dans la revendication 4, dans lequel l'ADN obtenu comprend moins de 2 % en poids de l'ARN présent dans le lysat.

6. Procédé tel que revendiqué dans la revendication 5, dans lequel l'ADN obtenu comprend moins de 1 % en poids de l'ARN présent dans le lysat.

7. Procédé tel que revendiqué dans l'une quelconque des revendications 1 à 6, dans lequel l'étape de précipitation au chlorure de calcium précède l'étape d'ultrafiltration.

8. Procédé tel que revendiqué dans l'une quelconque des revendications 1 à 6, dans lequel l'étape de précipitation au chlorure de calcium suit l'étape d'ultrafiltration.

9. Procédé tel que revendiqué dans l'une quelconque des revendications 1 à 8, dans lequel une ou plusieurs étapes de purification ou de polissage sont utilisées.

10. Procédé tel que revendiqué dans la revendication 9, dans lequel la ou les plusieurs étapes additionnelles de purification ou de polissage comprennent l'élimination d'une ou plusieurs des entités suivantes: endotoxines; protéines; ADN génomique et ARN.

11. Procédé tel que revendiqué dans la revendication 9 ou 10, dans lequel la ou les plusieurs étapes additionnelles de purification ou de polissage suivent l'étape d'ultrafiltration.

12. Procédé tel que revendiqué dans l'une quelconque des revendications 9, 10 ou 11, dans lequel la ou les plusieurs étapes de purification ou de polissage comprennent une étape de chromatographie.

13. Procédé tel que revendiqué dans la revendication 12, dans lequel l'étape de chromatographie est une étape de chromatographie par échange d'ions ou sur hydroxyapatite.

14. Procédé tel que revendiqué dans la revendication 13, dans lequel l'étape de chromatographie est une étape de chromatographie par échange d'ions.

15. Procédé tel que revendiqué dans la revendication 14, dans lequel l'étape de chromatographie par échange d'ions utilise la résine Fractogel TMAE.

16. Procédé tel que revendiqué dans l'une quelconque des revendications précédentes, dans lequel un précipité formé à l'étape de précipitation au chlorure de calcium est éliminé par une maille ou un tamis.

17. Procédé tel que revendiqué dans la revendication 16, dans lequel un précipité formé à l'étape de précipitation au chlorure de calcium est éliminé en même temps que les débris cellulaires par une maille ou un tamis.

18. Procédé tel que revendiqué dans l'une quelconque des revendications précédentes, dans lequel l'étape de précipitation au CaCl₂ élimine l'ARN de haut poids moléculaire et l'ADN génomique.

19. Procédé tel que revendiqué dans l'une quelconque des revendications 1-18, dans lequel l'étape d'ultrafiltration utilise une membrane avec un seuil de poids moléculaire inférieur à 500 KDa et l'ADN extrachromosomique est de 2 Kb à 200 Kb.

20. Procédé tel que revendiqué dans la revendication 19, dans lequel la membrane a un seuil de poids moléculaire inférieur à 300 KDa, plus préférablement d'environ 100 KDa.

21. Procédé tel que revendiqué dans la revendication 19, dans lequel la membrane est sélectionnée dans le groupe consistant en: polyéthersulfone; acétate de cellulose; polysulfone; polyvinylidène; polypropylène; polyamide; PES modifiée; polyvinylidine pyrolidine; fluorure de polyvinylidine (PVDF); nitrate de cellulose et triacétate de cellulose.

22. Procédé tel que revendiqué dans la revendication 21, dans lequel la membrane est une membrane polyéthersulfone.

23. Procédé tel que revendiqué dans l'une quelconque des revendications précédentes qui utilise un tampon de diafiltration ayant une plage de pH de 6-10.

24. Procédé tel que revendiqué dans la revendication 23, dans lequel le tampon de diafiltration est un tampon Tris.

25. Procédé tel que revendiqué dans la revendication 23, dans lequel le tampon de diafiltration est utilisé à plus de 10 volumes d'échanges.

26. Procédé tel que revendiqué dans la revendication 25, dans lequel le tampon de diafiltration est utilisé à plus de 20 volumes d'échanges.

27. Procédé tel que revendiqué dans la revendication 26, dans lequel le tampon de diafiltration est utilisé à plus de 30 volumes d'échanges.

28. Procédé tel que revendiqué dans la revendication 27, dans lequel le tampon de diafiltration est utilisé à plus de 40 volumes d'échanges.

29. Procédé tel que revendiqué dans la revendication 28, dans lequel le tampon de diafiltration est utilisé de 50 à 100 volumes d'échanges.

30. Procédé tel que revendiqué dans la revendication 19, dans lequel la membrane est utilisée avec un canal de criblage.

31. Procédé tel que revendiqué dans la revendication 30, dans lequel le canal de criblage est un canal en T.

32. Procédé tel que revendiqué dans l'une quelconque des revendications précédentes, où le procédé est effectué à une pression transmembranaire inférieure à 10 psi.

33. Procédé tel que revendiqué dans la revendication 19, dans lequel l'ADN extrachromosomique est chargé sur la membrane à une quantité inférieure à 30 mg/jt² dans le cas d'une membrane 300 K.

34. Procédé tel que revendiqué dans la revendication 13, dans lequel l'étape de chromatographie est une étape de chromatographie sur hydroxyapatite.

35. Procédé tel que revendiqué dans la revendication 34, dans lequel l'étape de chromatographie sur hydroxyapatite est effectuée à un pH de 7,3 à 8,3.

36. Procédé tel que revendiqué dans la revendication 35, dans lequel l'étape de chromatographie sur hydroxyapatite est effectuée à un pH compris entre 7,6 et 8,0.

37. Procédé tel que revendiqué dans la revendication 34, 35 ou 36, dans lequel l'hydroxyapatite est la Biorad Marcoprep Ceramic.

38. Procédé tel que revendiqué dans l'une quelconque des revendications 34 à 37, dans lequel un système de double tampon est utilisé pour séparer l'ADN extrachromosomique de l'ARN.

39. Procédé tel que revendiqué dans la revendication 38, dans lequel le système de double tampon comprend du Na₂HPO₄ pour éluer l'ARN et du K₂HPO₄ pour éluer l'ADN extrachromosomique.

40. Procédé tel que revendiqué dans la revendication 8, dans lequel l'étape de précipitation au CaCl₂ est suivie par une autre étape d'ultrafiltration ou une étape de désalinisation ou une étape de chromatographie en phase inverse.

41. Procédé tel que revendiqué dans la revendication 40, dans lequel l'étape d'ultrafiltration supplémentaire ou une étape de désalinisation ou une étape de chromatographie en phase inverse est suivie par l'étape de chromatographie.

42. Procédé tel que revendiqué dans la revendication 41, dans lequel l'étape de chromatographie est un échange d'ions.
